# EUROPEAN PATENT APPLICATION

(11) **EP 1 825 882 A1**
(43) Date of publication of application: **29.08.2007**
(21) Application number: 05816727.1
(22) Date of filing: 13.12.2005
(51) Int. Cl.: A61N 5/06

(54) **FINGERTIP STIMULATING DEVICE**

(30) Priority: 13.12.2004 JP 2004359454; 12.12.2005 JP 2005357189
(71) Applicant: Ekbo Inc., Atsugi-shi, Kanagawa 243-0212 (JP)
(72) Inventor: SUGIYAMA, Toshiki EKBO Inc., Kanagawa 243-0212 (JP); SHIMIZU, Yoshihiro EKBO Inc., Kanagawa 243-0212 (JP); ISHIWATA, Fumikazu, Kanegawa 259-1131 (JP)
(74) Representative: Grünecker, Kinkeldey, Stockmair & Schwanhäusser Anwaltssozietät
(86) International application number: PCT/JP2005/022887
(87) International publication number: WO 2006/064811

(57) **Abstract**

A fingertip stimulating apparatus that can be easily handled by any one, that can ensure stimulating a fingertip of a hand, and that can stabilize a body temperature of a person similarly to a body temperature of a healthy person by fingertip stimulation is provided. The fingertip stimulating apparatus (1) includes a fingertip arranging unit (3) on which the fingertip of the hand is arranged and a near-infrared generating member (4) irradiating a near-infrared ray onto a region near a border of a fingernail of the fingertip arranged on the fingertip arranging unit (3). A fingertip stimulating apparatus (300) includes fingertip arranging units (331) to (335) on which fingertips of the hand are arranged, respectively, near-infrared generating members (341) to (345) irradiating near-infrared rays onto regions near borders of fingernails of the respective fingertips arranged on the fingertip arranging units (331) to (345), and blue-violet light generating members (361) to (365) arranged visually recognizably and emitting blue-violet lights.

## Description

### TECHNICAL FIELD

The present invention relates to a fingertip stimulating apparatus for stimulating a fingertip of, for example, a hand.

### BACKGROUND ART

It is generally known that at a temperature of 37.2°C in a deep part of the body, enzymes essential to life sustaining activities function most actively. It is also known that average body temperatures are about 36.5°C to 36.7°C in the rectum and under the tongue and about 36.2°C to 36.3°C at armpits.

If a person is unable to maintain his or her body temperature that enables the body enzymes to actively function, activities of the enzymes naturally decline. The person may possibly become ill in some cases.

For a person with low blood pressure, for example, his or her body temperature changes to some extent in a day, i.e., the body temperature is lowest in the morning and gradually rises subsequently. While the body temperature of a healthy person is at least 35°C even in the morning, that of the person with low blood pressure is often as low as below 35°C in the morning.

Due to this, it is said that, for the person with low blood pressure, because of the low body temperature in the morning, body enzymes function insufficiently and that the phenomenon of energy deficiency occurs to the person. Namely, the person with low blood pressure cannot get up in the morning because the body enzymes function insufficiently due to low body temperature.

If such a state of low body temperature does not end in the morning but continues subsequently, this could cause a disease such as a cancer, a collagen disorder, an allergy or a gastric ulceration.

Conversely, if a person catches a high fever, it is said that brain cells of the person are destroyed. Actually, however, the brain cells are destroyed not by the high fever but the amount of oxygen necessary for the brain increases and the brain cells are destroyed because of insufficient supply of oxygen.

If the person catches a high fever, blood flow is constricted because the body turns into a state of tension. This results in poor blood circulation to the brain and eventual lack of oxygen in the brain. A child with a high fever falls into a fit of convulsions because of this lack of oxygen.

Moreover, "oversensitivity to cold" does not actually mean that the body of a person gets cold. When heat radiates from body surface due to air cooling or the like, autonomic nerves constrict blood vessels on the body surface and close person's pores in an environment in which the body surface is exposed to cold air so as not to give off the important heat from the deep part in the body. The body temperature is maintained deep in the body so as to avoid heat radiation as much as possible. This is why the person feels of cold.

In this way, a person always regulates the body temperature to be kept at 37.2°C in the deep part of the body. On hot days, a person sweats to radiate evaporation heat, thereby preventing the body temperature from excessively increasing. On cold days, the skin and blood vessels are constricted to thereby prevent the heat from being given off from the body. It is the "autonomic nerves" that are responsible for such control over the body temperature.

The autonomic nerves are nerves that unconsciously make all regulations in the body and include the sympathetic nerve and the parasympathetic nerve.

The sympathetic nerve acts to accelerate activities of the heart and breathing speed, to increase blood pressure, to increase blood flow, and to feed oxygen to all parts of the body for activities. On the contrary, the parasympathetic nerve acts to move the heart slowly and to relax the entire body. It can be considered that the sympathetic nerve mainly functions in the daytime and that the parasympathetic nerve mainly functions while the person is asleep.

The autonomic nerves regulate the activities of the heart and vasodilating and vasoconstrictive actions, and controls the blood pressure and blood flow. In alternatively expression, it is the "autonomic nerves" that cause each of the cells to decide whether to "function" or not. The autonomic nerves rule over almost "all" body cells and control cells suitable for an action at a certain time to function and unsuitable cells not to function.

When a person is excited, an instruction to activate the cells of the heart and blood vessels to make the body into an active state is issued from the sympathetic nerve. For example, when the person eats something, an instruction to activate the cells related to important digestion and absorption and to deactivate the other cells is issued from the parasympathetic nerve.

The blood circulating all around the body produces energy for maintaining the body temperature. When the person has a meal, the meal is digested and decomposed and converted into energy. Even when the person takes exercise, active muscles produce heat. The energy of the heat is distributed to all the cells in the body by the blood. Accordingly, cessation of the blood flow for some reason makes the blood supply insufficient and lowers the body temperature.

One of causes for the cessation of the blood flow is the tension of the sympathetic nerve. For example, when the person is busy working and deeply engaged in his or her work despite lack of sleep, the sympathetic nerve is continuously in a state of tension. Normally, however, even if the state of tension of the sympathetic nerve continues for a while, the parasympathetic nerve acts subsequently to return the body into an equilibrium state.

Nevertheless, if the person works too hard, the parasympathetic nerve cannot afford to act and the body is continuously tensed. Since the sympathetic nerve acts to constrict the blood vessels, the blood vessels of the person who works too hard become thinner. Because the amount of blood flowing in the thin vessels is small, the circulation volume of blood circulating all across the body decreases and the body temperature lowers.

Meanwhile, it is not necessarily true that the parasympathetic nerve always acts over the sympathetic nerve. Since the parasympathetic nerve causes dilation of blood vessels, the blood flows into the blood vessels in large amount. It takes lots of labor to move such a large amount of blood, resulting again in constricted blood flow.

From the aspect of the autonomic nerves, whether the sympathetic nerve or the parasympathetic nerve gains an advantage over the other, the body balance is disturbed, thereby lowering the body temperature to cause various diseases.

Moreover, in-vivo regulations such as a regulation of the amount of blood are not controlled solely by the autonomic nerves. The in-vivo regulations are made by interaction among three regulatory systems of the autonomic nerves, the endocrine system (hormonal secretion), and the "immune system". Therefore, the body temperature has a close relation not only with the autonomic nerves but also with the immune system.

If the sympathetic nerve is at advantage over the parasympathetic nerve, a ratio of lymph cells in white blood cells decreases. If the advantageous state of the sympathetic nerves continues and the body temperature further lowers, the ratio of lymph cells further decreases. This is because the lymph cells are under control of the parasympathetic nerve and granular leukocytes in the white blood cells are under control of the sympathetic nerve.

When the body temperature rises from the low temperature state, the parasympathetic nerve is advantageous over the sympathetic nerve this time and the ratio of lymph cells increases. However, too many lymph cells cause, in turn, the body temperature to lower. Because the low body temperature state is a state where the enzyme activities of enzymes are poor. This naturally makes the person at a risk of getting various types of diseases.

Stress makes the sympathetic nerve in a state of tension and causes low body temperature. The stress is a state where mental and physical burdens are imposed on a person, in which state it is necessary to feed a large amount of blood to the muscles throughout the body. Namely, the stress is a cause for making the sympathetic nerve excited.

The state of tense of the sympathetic nerve results not only from physical factors but also from mental agitations such as "worry", "anxiety", "amazement", "sorrow", and "irritation". Nevertheless, the parasympathetic nerve normally acts in place of the sympathetic nerve that is in the state of tense, so that the body balance is not disturbed.

However, if even weak stress continues for a long period of time, the parasympathetic nerve is often incapable of returning the body into the original state. If this happens, the sympathetic nerve gains an advantage over the parasympathetic nerve, resulting in low body temperature. According to persons who were taken seriously ill, they were under great stress without exception. Long lasting of the advantageous state of the sympathetic nerve places a person at risk.

It is "granular leukocytes" and "lymph cells" that assume the immune strength. The immune strength is an ability of processing bacteria and virus and toxic matters produced in the body and always maintaining the body in a state suitable for living. If the ability lowers, it induces various disorders.

Roughly speaking, the immune system consists of the granular leukocytes and the lymph cells. The importance aspect for allowing these immune cells to act most efficiently is the body temperature.

It is known that, if blood samples are taken from persons at body temperatures in a normal range (healthy persons) to examine states of the granular leukocytes and lymph cells, the person at a higher body temperature has more lymph cells. Because the lymph cells exclude matters that may do harm to a person, the more lymph cells can protect the body more strongly.

As described above, it is evident that the person at an average temperature equal to or lower than about 35°C, which average temperature is to be about 36.2°C to 36.3°C at armpits when the temperature is 37.2°C deep in the body, that is, "low body temperature" person has a temperature far lower than 37.2°C in the deep part of the body.

In case of such a "low body temperature" person, the "autonomic nerves" controlling the body temperature malfunction, the blood flow throughout the body tends to stagnate, and the "immune strength" possibly weakens. Compared to a person having a normal temperature, a person chronically at low body temperature is, in particular, lower in immune strength and more liable to diseases.

There is conventionally known methods of applying a physical stimulus such as "infrared radiation", "electric pulse" or "acupuncture" to a region considered to be most effective for the autonomic nerves so as to relieve the excited state of the sympathetic nerve or parasympathetic nerve of the autonomic nerves.

Moreover, it is understood that irradiation of an "optical pulse" onto the eyes can stabilize the state of the "autonomic nerves" (according to "Response of brain waves and peripheral blood flow to periodically blinking light stimulus", The School of Engineering, Tokai University).

Researchers of Tokai University asserts that the visual stimulation by light affects the "autonomic nerves" and has an effective result on the regulation of the "body temperature". This assertion is based on US research papers to the effect that many vibrators generating various spontaneous rhythms of frequency (rhythm generation sources) are present in the brain and interact with one another, thereby generating brain waves (cited from P.Andersen and S.A.A. Andersson: Physiological Basis of the Alpha Rhythm, Appleton-Century Crofts, N;Y., 1968).

Furthermore, while these vibrators usually act irregularly, they bond together, act synchronously, and generate a clear rhythm inducing brain waves when an internal stimulus resulting from the central nervous system or an external sensible stimulus is applied to the vibrators (from the study of Erol Basar: Brain Function and Oscillation, Springer, 1998, 1999).

The researches of Tokai University conducted a study about the effect of the response and light stimulation of the brain vibrators with respect to visual stimulation by a periodically blinking light on fluctuations in cardiovascular parameters such as heartbeat period and peripheral blood flow.

The study is none other than paying particular attention to the alpha pull-in effect of the blinking frequency of stimulating light among pull-in effects of the cerebral vibrators in response to the light stimulus, examining the relationship between the blinking frequency of the stimulating light and a change in the rhythm of regulating the body temperature inherent in the fluctuations in the heartbeat period and the fluctuations in peripheral blood flow, and considering the effect of the light stimulus on the activities of the autonomic nervous system.

First of all, the rise of the body temperature accompanies the rise in blood flow volume in peripheral blood vessels. Namely, when the body temperature rises, it is necessary that the heart beats at such a rhythm as to ensure a heart rate at which a sufficient blood flow volume can be maintained. The process of temperature rise includes a process of increasing the heart rate.

The role of the cardiopulmonary system is to maintain perfusion of the appropriately oxygenated blood to respective tissues and organs of the body. This operation is carefully regulated by the autonomic nervous system. The autonomic nervous system continuously perceives a blood pressure and an oxygen content of arteries, and changes breathing, cardiac outputs, and blood vessel resistance so as to keep the blood pressure and oxygen content of the arteries to fall within a narrow range. The autonomic nervous system acts as a feedback and control system in cooperation with effective organs regulated by the autonomic nervous system (according to Japanese Patent Publication No. H2-57933).

The present inventors expected from results of the above-stated study that the "stimulus of a pulsed light to the visual nerve" induces "the brain wave pull-in effect" and eventually changes peripheral vasomotion, and that the changed peripheral vasomotion changes blood pressure, causes regulations of the autonomic nervous system, and influences fluctuations (changes) in heart rate.

There is known the Kitney's study in 1975, "An Analysis of the Nonlinear Behaviour of the Human Thermal Vasomotor control System". In the study, analysis of a power spectral density of the fluctuations in peripheral blood flow made it clear that the human body temperature regulation system produces the rhythm at a period of about 20 seconds (to 0.05 Hz) in the blood flow of a fingertip via a change in vasomotion.

Moreover, according to the Akselrod's study in 1981, "Power Spectrum Analysis of Heart Rate Fluctuations; A Quantitative Probe of Beat-To-Beat Cardiovascular Control", a peak at about 0.05 Hz seen in the power spectral density of the heart rate fluctuations is a peak corresponding to a body temperature regulation rhythm at a period of about 20 seconds. Further, the peripheral vasomotion fluctuations cause a change in blood pressure, and reflect heart rate fluctuations through regulations of the autonomic nervous system.

Experiments conducted in Tokai University reveal that a light stimulus at 3 Hz causes greater peripheral vasomotion fluctuations than a stimulus at the other frequencies. Besides, the heart rate is "increased" by the light stimulus at 3 Hz and the increased state of the heart rate is maintained even after application of the stimulus.

This study concludes as follows. The central nervous system for the body temperature regulations is present in the hypothalamic area, and a light-based sensible stimulus influences the central nervous system. This induces excitation of the sympathetic nerve of the blood vessels. Further, a baroreflex feedback mechanism including the sympathetic nervous system and the renin-angiotensin system and accompanied by temporal delay causes vibration phenomena in an intensity change in a low frequency component of the peripheral blood flow fluctuations.

The "body-temperature regulation central nervous system" of interest is the "hypothalamic area" that is the center of the "autonomic nerves". The hypothalamic area is the center of the "sympathetic nerve" that promotes the heart function and the "parasympathetic nerve" that suppresses the heart function. At the same time, the hypothalamic area suppresses "body temperature", appetite, sleep, metabolism, and blood pressure.

The fact that the "light-based sensible stimulation" is applied to this region is equivalent to the stimulus of the light to the autonomic nervous center. Namely, the stimulus is considered to serve as a switch for the body temperature and the blood pressure regulations.

There is conventionally known "acupuncture" in Oriental medicine as a method of regulating the "autonomic nerves". An acupuncturist performs acupuncture on a proper trigger point of a patient after asking the patient about his or her disorder condition, thereby applying an effective "stimulus" to the autonomic nerves and easing the patient's disorder. It is known that the patient thereby effectively convalesces from the disorder.

There is also known a therapeutic apparatus employed for human body trigger point treatment as disclosed in Japanese Patent Application Laid-Open No. H3-244464.

### DISCLOSURE OF THE INVENTION

However, in case of the former, i.e., acupuncture, it is disadvantageously difficult for a nonprofessional person (non-acupuncturist) to perform acupuncture on himself or herself and particularly difficult to apply safe and effective "stimulus" to a trigger point.

In case of the latter, i.e., the therapeutic apparatus employed for human body trigger point treatment, a patient wears a treatment clothes with cut lines given in a certain range including positions of trigger points. It is disadvantageously difficult and inconvenient to deal with the clothes. Besides, it is disadvantageously unclear whether or not an effective "stimulus" can be applied to a trigger point, depending on how to wear or a magnitude of each cut line.

The present invention has been achieved to solve the above problems. It is an object of the present invention to provide a fingertip stimulating apparatus that can be easily handled by everybody, that can ensure stimulating a fingertip of a hand, that can apply a safe and effective stimulus to autonomic nerves by the fingertip stimulation, and that can control body temperature.

Another object of the present invention is to provide a fingertip stimulating apparatus that regulates body temperature to accelerate increase of peripheral blood flow by effectively providing a visual stimulation as well as a finger stimulation.

A first aspect of the present invention provides a fingertip stimulating apparatus that includes a fingertip arranging unit arranging a fingertip of a hand, and a near-infrared generating member irradiating a near-infrared ray onto a region near a border of a fingernail of the fingertip arranged on the fingertip arranging unit.

A second aspect of the present invention provides a fingertip stimulating apparatus that includes a fingertip arranging unit arranging a fingertip of a hand, a near-infrared generating member irradiating a near-infrared ray onto a region near a border of a fingernail of the fingertip arranged on the fingertip arranging unit, and a blue-violet light generating member arranged visually recognizably, and emitting a blue-violet light.

A third aspect of the present invention provides a fingertip stimulating apparatus that includes a fingertip arranging unit arranging a fingertip of a hand, a near-infrared generating member irradiating a near-infrared ray onto a region near a border of a fingernail of the fingertip arranged on the fingertip arranging unit, and a light pulse generating member arranged visually recognizably, and emitting a light pulse.

A fourth aspect of the present invention provides a fingertip stimulating apparatus that includes a fingertip arranging unit arranging a fingertip of a hand, a near-infrared generating member irradiating a near-infrared ray onto a region near a border of a fingernail of the fingertip arranged on the fingertip arranging unit, and a blue-violet light generating member irradiating a blue-violet light onto a ball or pad-side region of the fingertip arranged on the fingertip arranging unit.

### BRIEF DESCRIPTION OF THE DRAWINGS

[Fig. 1] Fig. 1 is a pattern view showing a first embodiment of a fingertip stimulating apparatus according to the present invention, and showing a fingertip from its side surface.
[Fig. 2] Fig. 2 is a view showing the fingertip from an arrow II direction shown in Fig. 1.
[Fig. 3] Fig. 3 is a view showing the fingertip from an arrow III direction shown in Fig. 1, and not showing a fingertip arranging unit.
[Fig. 4] Fig. 4 is a schematic exploded view showing a second embodiment of a fingertip stimulating apparatus according to the present invention.
[Fig. 5] Fig. 5 is a plan view of the second embodiment.
[Fig. 6] Fig. 6 is a front view of the second embodiment.
[Fig. 7] Fig. 7 is a bottom view of the second embodiment.
[Fig. 8] Fig. 8 is a cross-sectional plan view taken along a line IIX-IIX of Fig. 6.
[Fig. 9] Fig. 9 is a longitudinal side view taken along a line IX-IX of Fig. 5.
[Fig. 10] Fig. 10 is a circuit diagram showing one example of an electronic circuit.
[Fig. 11] Fig. 11 is an explanatory diagram showing an emission (irradiation) angle of a near-infrared LED.
[Fig. 12] Fig. 12 is an explanatory diagram showing arrangement angles if three near-infrared LEDs are employed.
[Fig. 13] Fig. 13 is an explanatory diagram showing an irradiation range of the near-infrared LEDs if the near-infrared LEDs are arranged as shown in Fig. 12.
[Fig. 14] Fig. 14 is a plan view of a cradle.
[Fig. 15] Fig. 15 is a front view of the cradle.
[Fig. 16] Fig. 16 is a side view of the cradle.
[Fig. 17] Fig. 17 is a front view showing a state of setting the fingertip stimulating apparatus to the cradle.
[Fig. 18] Fig. 18 is a side view showing the state of setting the fingertip stimulating apparatus to the cradle.
[Fig. 19] Fig. 19 is a cross-sectional plan view of principal parts showing a third embodiment of a fingertip stimulating apparatus according to the present invention.
[Fig. 20] Fig. 20 is a longitudinal side view of the principal parts of the third embodiment.
[Fig. 21] Fig. 21 is a cross-sectional plan view of principal parts showing a fourth embodiment of a fingertip stimulating apparatus according to the present invention.
[Fig. 22] Fig. 22 is a longitudinal side view of the principal parts of the fourth embodiment.
[Fig. 23] Fig. 23 is a graph showing a first experiment on a transition of body temperature of a person being tested obtained by irradiation of a near-infrared ray using the fingertip stimulating apparatus according to the second embodiment.
[Fig. 24] Fig. 24 shows a record of the body temperature transition of the first experiment.
[Fig. 25] Fig. 25 is a graph showing a second experiment on a transition of body temperature of a person being tested obtained by irradiation of a near-infrared ray using the fingertip stimulating apparatus according to the second embodiment.
[Fig. 26] Fig. 26 shows a record of the body temperature transition of the second experiment.
[Fig. 27] Fig. 27 is a graph showing a third experiment on a transition of body temperature of a person being tested obtained by irradiation of a near-infrared ray using the fingertip stimulating apparatus according to the second embodiment.
[Fig. 28] Fig. 28 shows a record of the body temperature transition of the third experiment.
[Fig. 29] Fig. 29 is a graph showing a fourth experiment on a transition of body temperature of a person being tested obtained by irradiation of a near-infrared ray using the fingertip stimulating apparatus according to the second embodiment.
[Fig. 30] Fig. 30 shows a record of the body temperature transition of the fourth experiment.
[Fig. 31] Fig. 31 is a graph showing an intensity distribution of an emission spectrum of a fluorescent lamp in a band of 400 nm.
[Fig. 32] Fig. 32 is a front view showing a fifth embodiment of a fingertip stimulating apparatus according to the present invention.
[Fig. 33] Fig. 33 is a plan view showing the fifth embodiment.
[Fig. 34] Fig. 34 is a plan view showing arrangement of fingertip insertion units.
[Fig. 35] Fig. 35 is a longitudinal sectional view taken along a line XXXV-XXXV of Fig. 34.
[Fig. 36] Fig. 36 is a longitudinal sectional view taken along a line XXXVI-XXXVI of Fig. 34.
[Fig. 37] Fig. 37 is a plan view showing a support plate.
[Fig. 38] Fig. 38(a) is plan view showing a lower support plate and Fig. 38 (b) is a front view showing the lower support plate.
[Fig. 39] Fig. 39 is a plan view showing an upper support plate.
[Fig. 40] Fig. 40 is a plan view showing a cushion member.
[Fig. 41] Fig. 41(a) is a plan view showing LED units and Fig. 41(b) is a front view showing the LED units.
[Fig. 42] Fig. 42(a) is a longitudinal sectional view showing a state of arranging a pin in the fingertip insertion unit shown in Fig. 35, and Fig. 42(b) is a longitudinal sectional view showing a state of arranging the LED unit in the fingertip insertion unit shown in Fig. 35.
[Fig. 43] Fig. 43 is a longitudinal sectional view showing a state of arranging the LED unit in the fingertip insertion unit shown in Fig. 36.
[Fig. 44] Fig. 44 is a plan view showing a state of arranging LED units in respective fingertip insertion units.
[Fig. 45] Fig. 45 is a front view showing a state of arranging LED units in respective fingertip insertion units.
[Fig. 46] Fig. 46 is a front view showing a state of arranging the cushion member.
[Fig. 47] Fig. 47 is an exploded front view of a fifth embodiment.
[Fig. 48] Fig. 48 is an assembly diagram showing an interior of the fifth embodiment.
[Fig. 49] Fig. 49 is a block diagram of the fifth embodiment. [Fig. 50] Fig. 50 is a graph showing a first experiment conducted to a person being tested about transition in basal metabolism using the fingertip stimulating apparatus according to the fifth embodiment.
[Fig. 51] Fig. 51 is a graph showing a second experiment conducted to a person being tested about transition in basal metabolism using the fingertip stimulating apparatus according to the fifth embodiment.
[Fig. 52] Fig. 52 is a graph showing a third experiment conducted to a person being tested about transition in basal metabolism using the fingertip stimulating apparatus according to the fifth embodiment.
[Fig. 53] Fig. 53 is a graph showing a fourth experiment conducted to a person being tested about transition in basal metabolism using the fingertip stimulating apparatus according to the fifth embodiment.
[Fig. 54] Fig. 54 is a graph showing a fifth experiment conducted to a person being tested about transition in basal metabolism using the fingertip stimulating apparatus according to the fifth embodiment.
[Fig. 55] Fig. 55 is a graph showing a sixth experiment conducted to a person being tested about transition in basal metabolism using the fingertip stimulating apparatus according to the fifth embodiment.
[Fig. 56] Fig. 56 is a graph showing a seventh experiment conducted to a person being tested about transition in basal metabolism using the fingertip stimulating apparatus according to the fifth embodiment.
[Fig. 57] Fig. 57 is a plan view showing a sixth embodiment of a fingertip stimulating apparatus according to the present invention.
[Fig. 58] Fig. 58 is a front view of principal parts showing the sixth embodiment.
[Fig. 59] Fig. 59 is a longitudinal sectional view taken along a line LIX-LIX of Fig. 57.
[Fig. 60] Fig. 60 is a longitudinal sectional view taken along a line LX-LX of Fig. 57.
[Fig. 61] Fig. 61 is a schematic longitudinal sectional view showing an exploded view of a seventh embodiment of a fingertip stimulating apparatus according to the present invention.
[Fig. 62] Fig. 62 is a schematic longitudinal sectional view of a cradle employed in the seventh embodiment.
[Fig. 63] Fig. 63 is an exploded view showing the seventh embodiment.
[Fig. 64] Fig. 64 is a perspective view of a main housing.
[Fig. 65] Fig. 65 is a partially cross-sectional perspective view of a fingertip insertion unit.
[Fig. 66] Fig. 66 is a cross-sectional view of the fingertip insertion unit.
[Fig. 67] Fig. 67 is a cross-sectional view showing a state of fixing a rubber fingerstall to a fingertip insertion unit.
[Fig. 68] Fig. 68 (a) is a perspective view showing a state of attaching LED unit to the main housing from one direction, Fig. 68 (b) is a perspective view showing the state of attaching the LED unit to the main housing from the other direction, and Fig. 68 (c) is a schematic plan view showing the state of attaching the LED unit to the main housing.
[Fig. 69] Fig. 69 is a cross-sectional view of principal parts schematically showing the seventh embodiment.
[Fig. 70] Fig. 70(a) is a plan view showing an example of an open/close mechanism, and Fig. 70(b) is an enlarged view of a part A.
[Fig. 71] Fig. 71 (a) is a front view showing a rotation plate, and Fig. 71 (b) is a perspective view of principal parts showing the rotation plate.
[Fig. 72] Fig. 72 is a plan view of principal parts showing a state where the open/close mechanism operates.
[Fig. 73] Fig. 73 (a) is a plan view showing another example of the open/close mechanism, and Fig. 73(b) is an enlarged view of a part B.
[Fig. 74] Fig. 74(a) is a perspective view of principal parts showing a relationship between a lock ring and a lock pin, and Fig. 74(b) is a cross-sectional view showing the relationship between the lock ring and the lock pin.
[Fig. 75] Fig. 75(a) is a perspective view showing a state where the lock pin is at a lock position, and Fig. 75(b) is a cross-sectional view showing the state where the lock pin is at the lock position.
[Fig. 76] Fig. 76 (a) is a perspective view showing a state where the lock pin is at a unlock position, and Fig. 76(b) is a cross-sectional view showing the state where the lock pin is at the unlock position.
[Fig. 77] Fig. 77 is a plan view showing a principle of an eighth embodiment of a fingertip stimulating apparatus according to the present invention.
[Fig. 78] Fig. 78(a) is a perspective view showing the principle of the eighth embodiment, and Fig. 78(b) is a perspective view showing an application of the eighth embodiment.
[Fig. 79] Fig. 79 is a plan view showing the principle if the eighth embodiment is applied to five fingers.
[Fig. 80] Fig. 80(a) is a pattern diagram showing direct irradiation by a near-infrared LED, and Fig. 80 (b) is a pattern diagram showing reflection of irradiation by the near-infrared LED.
[Fig. 81] Fig. 81 is an explanatory diagram showing a ninth embodiment of a fingertip stimulating apparatus according to the present invention.

### BEST MODE FOR CARRYING OUT THE INVENTION

Embodiments of the present invention are explained below in detail with reference to the drawings.

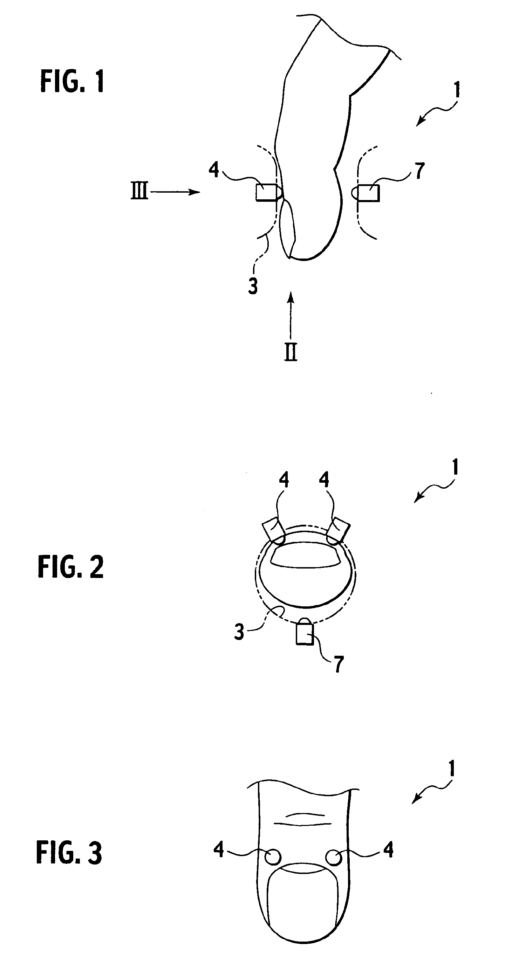

Figs. 1 to 3 are pattern diagrams showing a first embodiment of a fingertip stimulating apparatus according to the present invention. Fig. 1 is a side view of a fingertip, Fig. 2 is a view showing the fingertip from an arrow II direction shown in Fig. 1, and Fig. 3 is a view showing the fingertip from an arrow III direction shown in Fig. 1. A fingertip arranging unit is not shown in Figs. 1 to 3.

A fingertip stimulating apparatus 1 shown therein includes a fingertip arranging unit 3 on which at least one fingertip (preferably all fingertips of one hand) is arranged. A near-infrared generating member 4 that irradiates a near-infrared ray onto a region near a border of a fingernail of the arranged fingertip is provided on this fingertip arranging unit 3.

A wavelength of the near-infrared ray which the near-infrared generating member 4 irradiates onto the region near the border of the fingernail of the fingertip is preferably in a range of 820 nm to 920 nm, more preferably near 870 nm. Such a near-infrared generating member 4 can be constituted by at least one near-infrared LED (two in the drawings).

It is preferable that such a near-infrared generating member 4 irradiates the near-infrared ray onto the region near the border of the fingernail of the fingertip while contacting it. It is also preferable that the near-infrared generating member 4 irradiates the near-infrared ray onto the region while intermittently turning on or off the near-infrared ray.

The fingertip stimulating apparatus 1 also includes a blue-violet light generating member 7 provided on the fingertip arranging unit 3 and irradiating a blue-violet light onto a ball or pad-side region of the fingertip arranged on the fingertip arranging unit 3.

A wavelength of the blue-violet light which the blue-violet light generating member 7 irradiates onto the ball or pad-side region of the fingertip is preferably in a range of 390 nm to 420 nm, more preferably near 405 nm. Such a blue-violet light generating member 7 can be constituted by a blue-violet LED.

Various specific designs can be applied to the fingertip stimulating apparatus 1. Some of design examples will be shown below and embodiments of the design examples will be described.

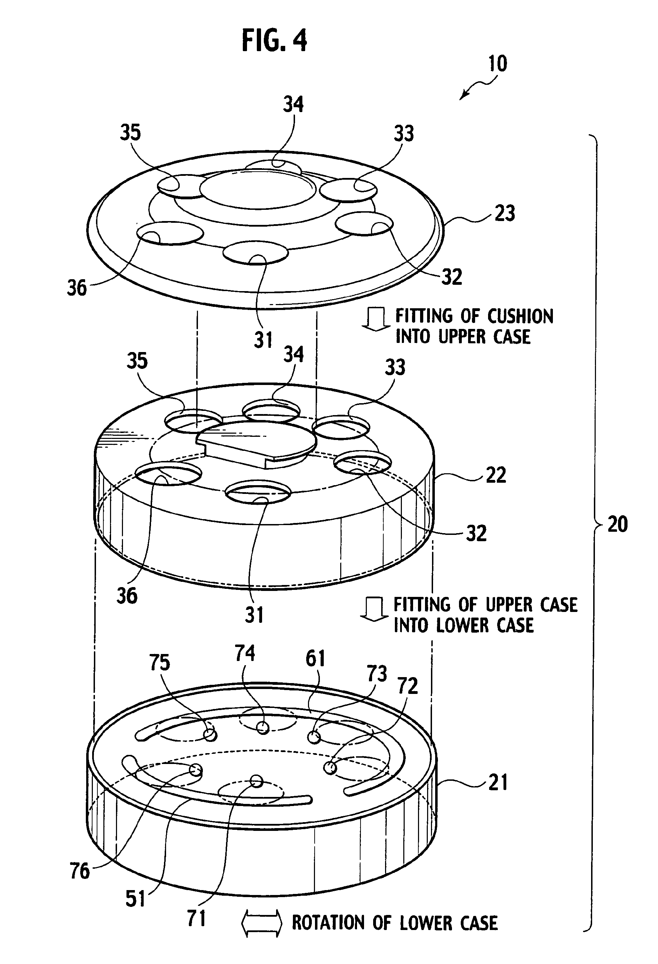

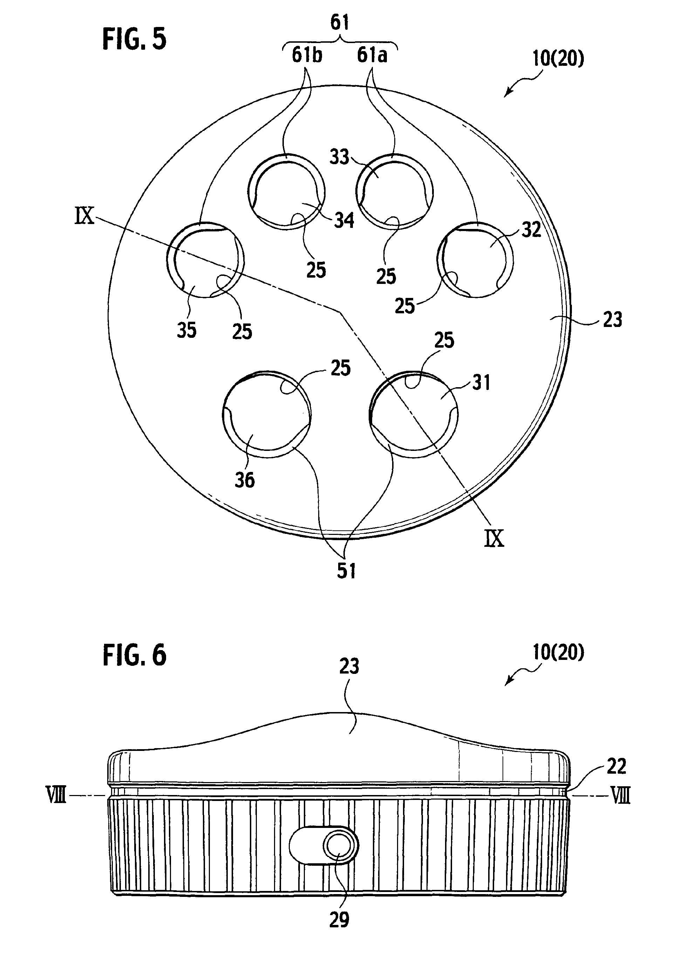

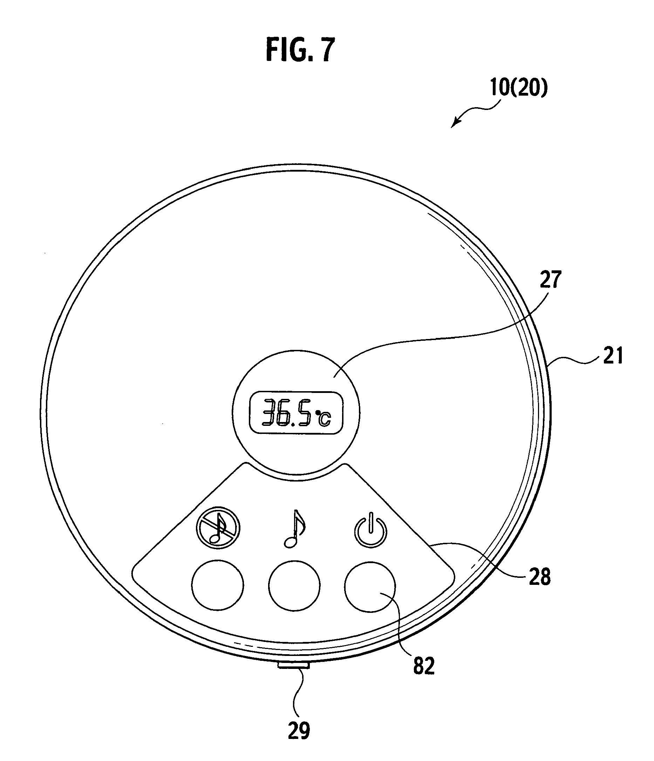

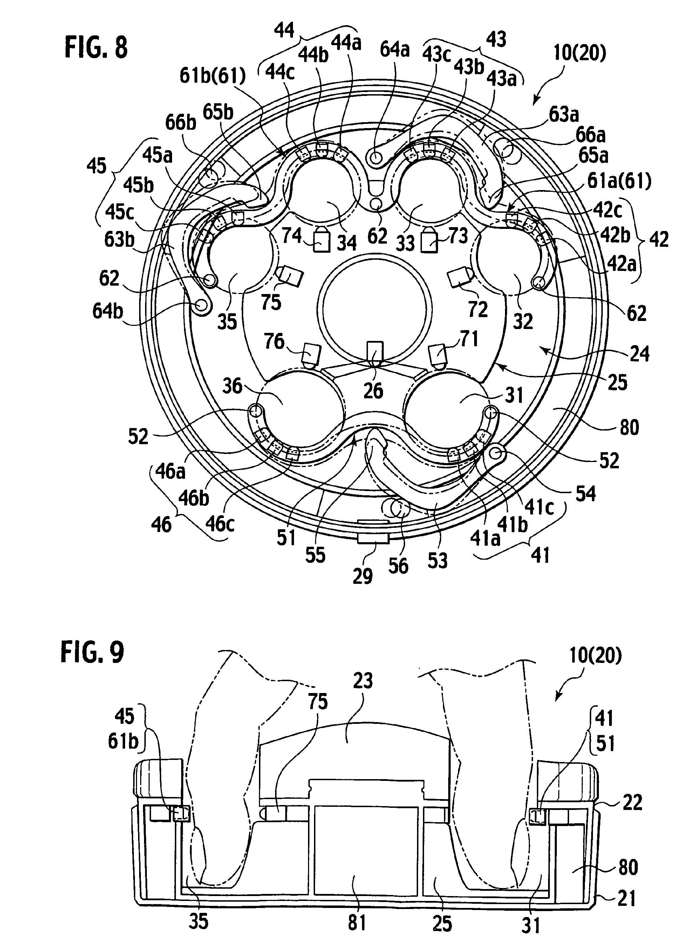

Figs. 4 to 9 show a second embodiment of the fingertip stimulating apparatus according to the present invention. Fig. 4 is a schematic exploded view, Fig. 5 is a plan view, Fig. 6 is a front view, Fig. 7 is a bottom view, Fig. 8 is a cross-sectional plan view taken along a line IIX-IIX of Fig. 6, and Fig. 9 is a longitudinal side view taken along a line IX-IX of Fig. 5.

A fingertip stimulating apparatus 10 shown therein is configured so that a substantially disk-shaped apparatus main body 20 having a required thickness to insert fingertips into the apparatus main body 20 includes six fingertip insertion units 31, 32, 33, 34, 35, and 36.

Among them, the fingertip insertion units 31, 32, 33, 34, and 35 are units into which a first finger (thumb) L1, a second finger (forefinger) L2, a third finger (long finger) L3, a fourth finger (medical finger) L4, and a fifth finger (little finger) L5 of a left hand are inserted, respectively.

The fingertip insertion units 36, 35, 34, 33, and 32 are units into which a first finger (thumb) R1, a second finger (forefinger) R2, a third finger (long finger) R3, a fourth finger (medical finger) R4, and a fifth finger (little finger) R5 of a right hand are inserted, respectively.

Due to this, the fingertip insertion unit 31 is independent unit for the first finger (thumb) L1 of the left hand, and the fingertip insertion unit 36 is independent unit for the first finger (thumb) R1 of the right hand.

On the other hand, the fingertip insertion unit 32 is shared between the second finger (forefinger) L2 of the left hand and the fifth finger (little finger) R5 of the right hand.

Further, the fingertip insertion unit 33 is shared between the third finger (long finger) L3 of the left hand and the fourth finger (medical finger) R4 of the right hand.

The fingertip insertion unit 34 is shared between the fourth finger (medical finger) L4 of the left hand and the third finger (long finger) R3 of the right hand.

Moreover, the fingertip insertion unit 35 is shared between the fifth finger (little finger) L5 of the left hand and the second finger (forefinger) R2 of the right hand.

The apparatus main body 20 is configured to include a lower case 21 having a bottom, an upper case fitted into the lower case 21, and a cushion member 23 arranged on an upper surface of the upper case 22.

The upper case 22 includes a main space unit 24 in which six holes for inserting the fingertips are formed on its upper surface (see Fig. 8).

In this main space unit 24, a finger pad member 25 made of an appropriate material that is filled with a matter, e.g. , a gel agent, smoothing to the touch for the fingertips, thereby forming outlines of at least fingertip ball or pad-sides of the six fingertip insertion units 31, 32, 33, 34, 35, and 36 is arranged (see Figs. 8 and 9).

The main space unit 24 is designed to have a depth so that neighbors of top joints (joints of forefront end) including at least entire fingernails of the respective fingers are sunk into the main space unit 24 when the fingertips of the five fingers of the left hand are inserted into the respective fingertip insertion units 31 to 35 or those of the five fingers of the right hand are inserted into the respective fingertip insertion units 36 to 32 and contact with a bottom of the finger pad member 25 (see Fig. 9).

Further, near-infrared LEDs 41, 42, 43, 44, 45, and 46 irradiating near-infrared rays onto the regions near the borders of fingernails are arranged on fingertip back-sides (nail-sides) of the six fingertip insertion units 31, 32, 33, 34, 35, and 36 at predetermined heights, respectively.

Each of these near-infrared LEDs are constituted by three near-infrared LEDs. Namely, the near-infrared LEDs 41, 42, 43, 44, 45, and 46 are configured to include three near-infrared LEDs 41a, 41b, and 41c, three near-infrared LEDs 42a, 42b, and 42c, three near-infrared LEDs 43a, 43b, and 43c, three near-infrared LEDs 44a, 44b, and 44c, three near-infrared LEDs 45a, 45b, and 4 5c , and three near-infrared LEDs 46a, 46b, and 46c, respectively.

Among them, the near-infrared LEDs 41a, 41b, and 41c on the fingertip insertion unit 31 side and the near-infrared LEDs 46a, 46b, and 46c on the fingertip insertion unit 36 side are attached to one flexible band to constitute a first LED band 51.

The first LED band 51 has both ends fixed to the upper case 22 by fixing pins 52, 52 respectively. The other end 55 of a band pressure cam 53 having one end rotatably attached to the upper case 22 by a pivotal pin 54 presses a central portion of the first LED band 51 inward.

The first LED band 51 is configured so that the central portion thereof is pressed inward by causing a cam drive rod 56 fixed to the lower case 21 to drive the band pressure cam 53 inward by rotations of the lower case 21 and the upper case 22 relative to each other.

The near-infrared LEDs on the other fingertip insertion unit 32 to 35 sides are attached to another flexible band, thereby constituting a second LED band 61.

Both ends and a central portion of the second LED band 61 are fixed to the upper case 22 by fixing pins 62, whereby the second LED band 61 is divided into a band movable unit 61a, to which the near-infrared LEDs 42a, 42b, and 42c on the fingertip insertion unit 32 side and the near-infrared LEDs 43a, 43b, and 43c on the fingertip insertion unit 33 side are attached, and a band movable unit 61b, to which the near-infrared LEDs 44a, 424b, and 44c on the fingertip insertion unit 34 side and the near-infrared LEDs 45a, 45b, and 45c on the fingertip insertion unit 35 side are attached.

A central portion of the band movable unit 61a of the second LED band 61 is pressed inward by the other end 65a of a band pressure cam 63a having one end rotatably attached to the upper case 22 by a pivotal pin 64a.

The band movable unit 61a is configured so that the central portion thereof is pressed inward by causing a cam drive rod 66a fixed to the lower case 21 to drive the band pressure cam 63a inward by the rotation of the lower case 21 and the upper case 22 relative to each other.

Likewise, a central portion of the band movable unit 61b of the second LED band 61 is pressed inward by the other end 65b of the band pressure cam 63b having one end rotatably attached to the upper case 22 by a pivotal pin 64b.

The band movable unit 61b is configured so that the central portion thereof is pressed inward by causing a cam drive rod 66b fixed to the lower case 21 to drive the band pressure cam 63b inward by the rotation of the lower case 21 and the upper case 22 relative to each other.

Furthermore, in the main space unit 24 of the apparatus main body 2, blue-violet LEDs 71, 72, 73, 74, 75, and 76 irradiating blue-violet lights onto ball or pad-side regions of fingertips are arranged on fingertip ball or pad-sides (opposite to nail-sides) of the six fingertip insertion unit 31, 32, 33, 34, 35, and 36 at predetermined heights, respectively.

Moreover, a temperature sensor 26 that detects a temperature of, for example, a ball or pad side of the first finger (thumb) L1 of the left hand inserted into the finger insertion unit 31 or that of the first finger (thumb) R1 of the right hand inserted into the finger insertion unit 36 is provided in the apparatus main body 20.

The temperature detected by the temperature sensor 26 is displayed on a temperature display unit 27 arranged on a bottom of the lower case 21, as shown in Fig. 7.

As shown in Fig. 7, an operation panel 28 of the fingertip stimulating apparatus 10 (apparatus main body 20) is provided on the bottom of the lower case 21. An AC adapter jack 29 is provided on a side surface of the apparatus main body 20.

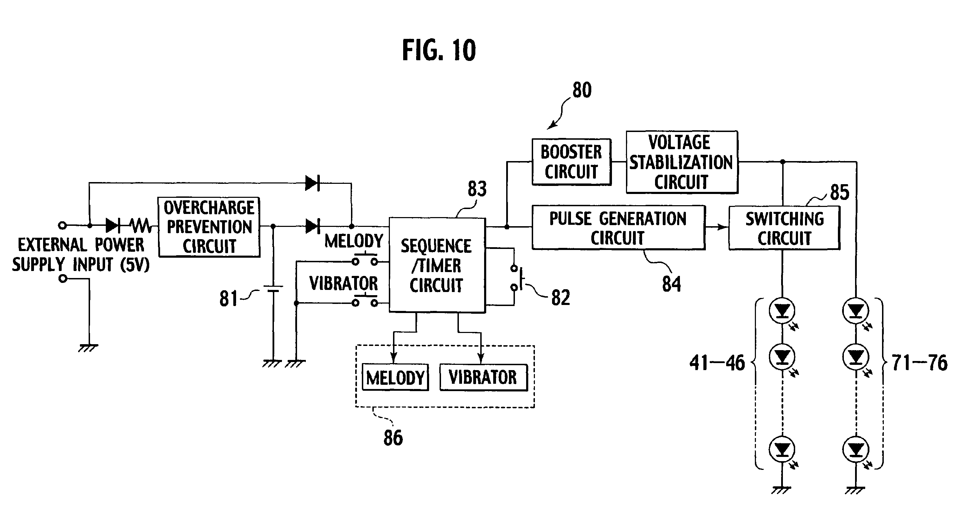

An electronic circuit 80 is arranged in the apparatus main body 20 at an appropriate position outside of the main space unit 24, and electrically connected to a battery 81 arranged at an appropriate position inside of the main space unit 24. Fig. 10 shows one example of the electronic circuit 80.

As shown in Fig. 10, the electronic circuit 80 includes a sequence/timer circuit 83 to which a power switch 82 is connected. The electronic circuit 80 carries current across the near-infrared LEDs 41 to 46 and the blue-violet LEDs 71 to 76 according to conditions set to the sequence/timer circuit 83, thereby causing the near-infrared LEDs 41 to 46 and the blue-violet LEDs 71 to 76 to emit and irradiate rays or lights.

A pulse generation circuit 84 and a switching circuit 85 are provided in a power supply circuit for the near-infrared LEDs 41 to 46 among the LEDs 41 to 46 and 71 to 76. Due to this, the near-infrared LEDs 41 to 46 are repeatedly turned on or off in a predetermined period.

When an abnormality deviating from the conditions set to the sequence/timer circuit 83 occurs, an alarm circuit 86 by either a melody or a vibrator selected and set in advance starts operating to alarm a user.

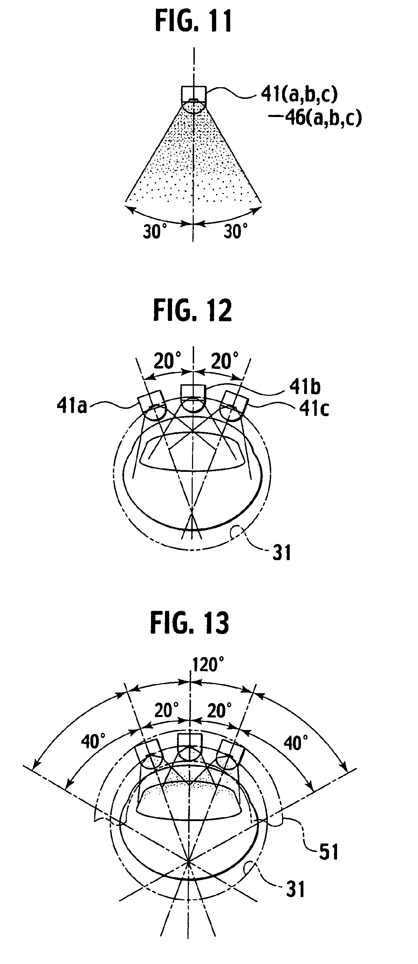

Fig. 11 is an explanatory diagram showing an emission (irradiation) angle of one of the near-infrared LEDs 41 (a, b, c) to 46 (a, b, c). Fig. 12 is an explanatory diagram showing arrangement angles at which the three near-infrared LEDs 41a, 41b, and 41c constituting each of the near-infrared LEDs (e.g., near- infrared LED41) are arranged. Fig. 13 is an explanatory diagram showing an irradiation range as a result of arranging of the near-infrared LEDs 41a, 41b, and 41c.

As shown in Fig. 11, the emission (irradiation) angle of one near-infrared LED (e.g., 41a) is 30° about an optical axis. If three of such near-infrared LEDs (e.g., 41a, 41b, and 41c) are laid side by side and arranged at an angle of 20° relative to one of the other LEDs as shown in Fig. 12, the overall irradiation range has an angle of 120° as shown in Fig. 13.

When the above-stated fingertip stimulating apparatus 10 is used, the first finger (thumb) L1, the second finger (forefinger) L2, the third finger (long finger) L3, the fourth finger (medical finger) L4, and the fifth finger (little finger) L5 of the left hand are inserted into the respective fingertip insertion units 31, 32, 33, 34, and 35 or the first finger (thumb) R1, the second finger (forefinger) R2, the third finger (long finger) R3, the fourth finger (medical finger) R4, and the fifth finger (little finger) R5 of the right hand are inserted into the respective fingertip insertion units 36, 35, 34, 33, and 32.

If the user holds the side surface of the lower case 21 by the hand the fingers of which are not inserted and slightly rotates the lower case 21 toward the upper case 22 into which the fingers are inserted, the cam drive rod 56 drives the band pressure cam 53 inward, thereby pressing the central portion of the first LED band 51 inward. Further, the cam drive rods 66a and 66b drive the band pressure cams 63a and 63b inward, thereby pressing the central portions of the band movable units 61a and 61b of the second LED band 61 inward, respectively.

By pressing the central portion of the first LED band 51 inward, the near-infrared LEDs 41a, 41b, and 41c attached to the LED band 51 touch and lightly press the region near the border of the fingernail of the first finger (thumb) L1 of the left hand, or the near-infrared LEDs 46a, 46b, and 46c attached to the LED band 51 touch and lightly press the region near the border of the fingernail of the first finger (thumb) R1 of the right hand.

Furthermore, by pressing the central portion of the band movable unit 61a of the second LED band 61 inward, the near-infrared LEDs 42a, 42b, and 42c and 43a, 43b, and 43c attached to the band movable unit 61a touch and lightly press the regions near the borders of the fingernails of the second finger (forefinger) L2 and the third finger (long finger) L3 of the left hand or the regions near the borders of the fingernails of the fourth finger (medical finger) R4 and the fifth finger (little finger) R5 of the right hand.

Likewise, by pressing the central portion of the band movable unit 61b of the second LED band 61 inward, the near-infrared LEDs 44a, 44b, and 44c and 45a, 45b, and 45c attached to the band movable unit 61b touch and lightly press the regions near the borders of the fingernails of the fourth finger (medical finger) L4 and the fifth finger (little finger) L5 of the left hand or the regions near the borders of the fingernails of the second finger (forefinger) R2 and the third finger (long finger) R3 of the right hand.

If the power switch 82 on the operation panel 28 is turned on in this state, then the sequence/timer circuit 83 starts and carries current across the near-infrared LEDs 41 to 46 and the blue-violet LEDs 71 to 76 according to the set conditions.

Among the LEDs, the near-infrared LEDs 41 to 46 are repeatedly turned on or off in a predetermined period during timer set time by functions of the pulse generation circuit 84 and the switching circuit 85. As a result, the near-infrared rays are intermittently irradiated on the regions near the borders of the fingernails of the five fingers L1 to L5 of the left hand inserted into the respective fingertip insertion units 31 to 35 or those of the five fingers R1 to R5 of the right hand inserted into the respective fingertip insertion units 36 to 32.

On the other hand, the blue-violet LEDs 71 to 76 continue to be turned on during timer set time. As a result, blue-violet lights are continuously irradiated onto the fingertip ball or pad-side regions of the five fingers L1 to L5 of the left hand inserted into the respective fingertip insertion units 31 to 35 or those of the five fingers R1 to R5 of the right hand inserted into the respective fingertip insertion units 36 to 32.

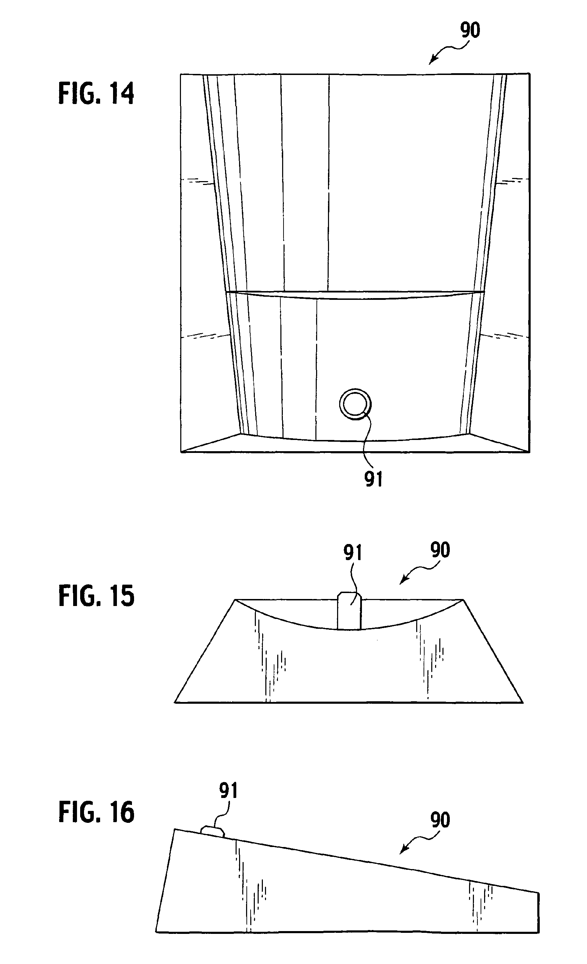

Fig. 14 is a plan view of a cradle to which the apparatus main body 20 is set when the fingertip stimulating apparatus 10 is not used. Fig. 15 is a front view of the cradle and Fig. 16 is a side view of the cradle. A cradle 90 shown therein is connectable to an AC power supply source such as an outlet using a power cord (not shown). The cradle 90 includes a main body connection AC jack 91.

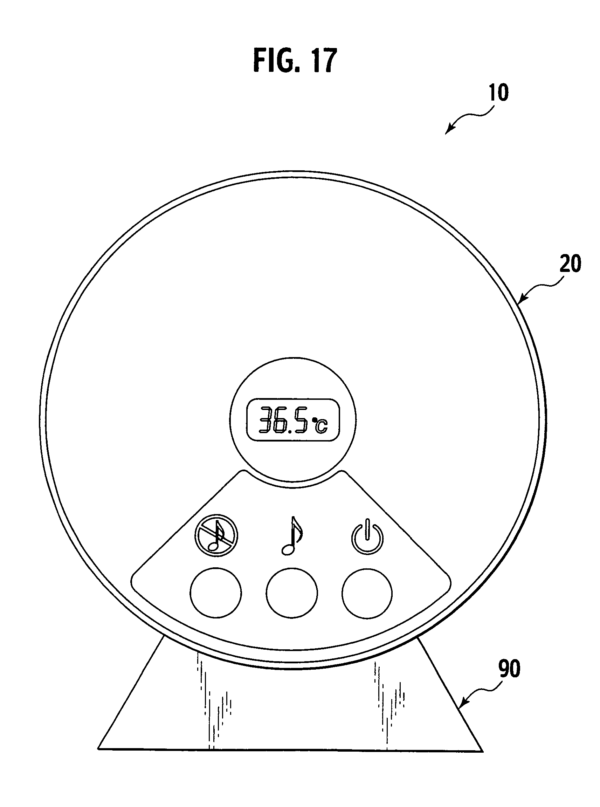

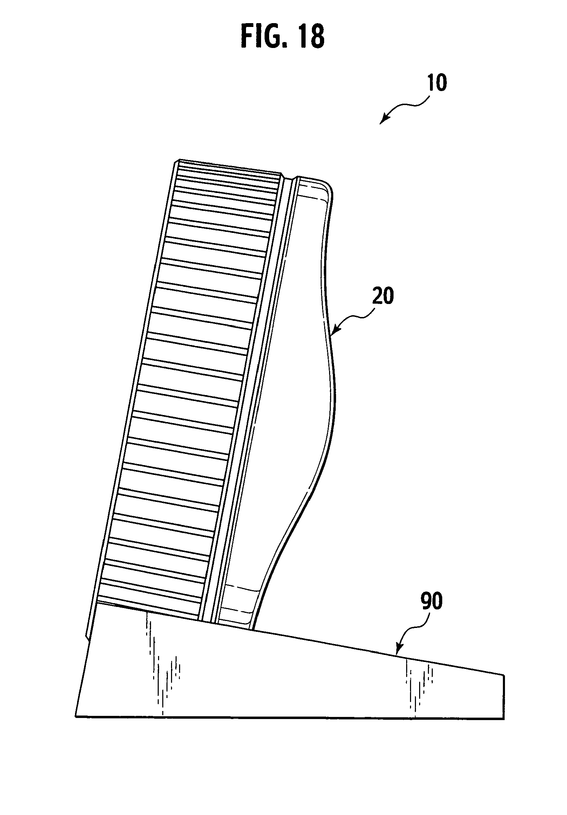

When the apparatus main body 20 of the fingertip stimulating apparatus 10 is set to the cradle 90, the AC adapter jack 29 is positioned so as to be fitted into the main body connection AC jack 91 of the cradle 90. Figs. 17 and 18 show a state of setting the apparatus main body 20 to the cradle 90.

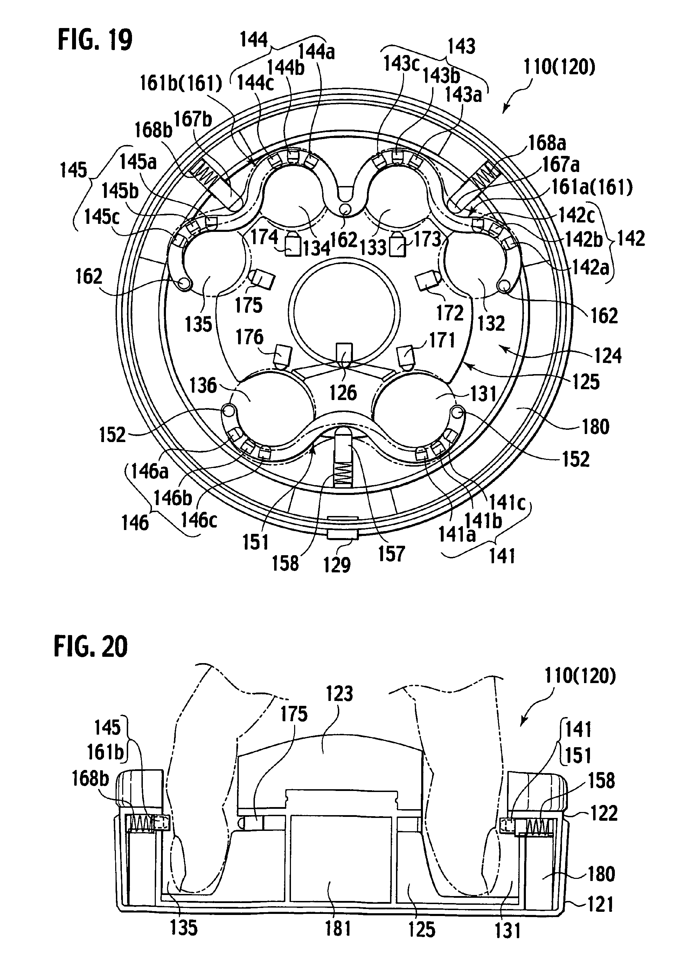

Figs. 19 and 20 show a third embodiment of the fingertip stimulating apparatus according to the present invention. Fig. 19 is a cross-sectional plan view (corresponding to Fig. 8) of principal parts, and Fig. 20 is a longitudinal side view (corresponding to Fig. 9) of the principal parts.

A fingertip stimulating apparatus 110 shown therein is almost similar to the fingertip stimulating apparatus 10 shown in Figs. 4 to 9 (and Figs. 10 to 18). Therefore, like constituent elements are denoted by reference numerals that are like reference numerals plus 100 as those used in the fingertip stimulating apparatus 10, and detailed descriptions and illustrations thereof will be omitted.

The fingertip stimulating apparatus 110 differs from the fingertip stimulating apparatus 10 in a structure of pressing a central portion of a first LED band 151 inward and a structure of pressing central portions of movable units 161a and 161b of a second LED band 161 inward.

Namely, the fingertip stimulating apparatus 110 is configured so that a band forcing member 157 corresponding to the central portion of the first LED band 151, a band forcing member 167a corresponding to the central portion of the band movable unit 161a of the second LED band 161, and a band forcing member 167b corresponding to the central portion of the band movable unit 161b of the second LED band 161 are provided in an upper case 122.

The band forcing member 157 includes a compression spring 158 and is biased by the compression spring 158 inward of a radial direction of an apparatus main body 120. Due to this, the band forcing member 157 is configured to press the central portion of the first LED band 151 inward by the biasing force.

The band forcing member 167a includes a compression spring 168a and is biased by the compression spring 168a inward of the radial direction of the apparatus main body 120. Due to this, the band forcing member 167a is configured to press the central portion of the band movable unit 161a of the second LED band 161 inward by the bias force.

Likewise, the band forcing member 167b includes a compression spring 168b and is biased by the compression spring 168b inward of the radial direction of the apparatus main body 120. Due to this, the band forcing member 167b is configured to press the central portion of the band movable unit 161b of the second LED band 161 inward by the bias force.

When the above-stated fingertip stimulating apparatus 110 is used, the first finger (thumb) L1, the second finger (forefinger) L2, the third finger (long finger) L3, the fourth finger (medical finger) L4, and the fifth finger (little finger) L5 of the left hand are inserted into respective fingertip insertion units 131, 132, 133, 134, and 135 or the first finger (thumb) R1, the second finger (forefinger) R2, the third finger (long finger) R3, the fourth finger (medical finger) R4, and the fifth finger (little finger) R5 of the right hand are inserted into respective fingertip insertion units 136, 135, 134, 133, and 132.

The central portion of the first LED band 151 is pressed inward by the band forcing member 157. Due to this, by inserting the fingertips, near-infrared LEDs 141a, 141b, and 141c attached to the first LED band 151 touch and lightly press the region near the border of the fingernail of the first finger (thumb) L1 of the left hand, or near-infrared LEDs 146a, 146b, and 146c attached to the first LED band 151 touch and lightly press the region near the border of the fingernail of the first finger (thumb) R1 of the right hand.

Furthermore, the central portion of the band movable unit 161a of the second LED band 161 is pressed inward by the band forcing member 167a. Due to this, by inserting the fingertips, near-infrared LEDs 142a, 142b, and 142c attached to the band movable unit 161a touch and lightly press the region near the borders of the fingernails of the second finger (forefinger) L2 and the third finger (long finger) L3 of the left hand or the regions near the borders of the fingernails of the fourth finger (medical finger) R4 and the fifth finger (little finger) R5 of the right hand.

Likewise, the central portion of the band movable unit 161b of the second LED band 161 is pressed inward by the band forcing member 167b. Due to this, by inserting the fingertips, near-infrared LEDs 144a, 144b, and 144c attached to the band movable unit 161b touch and lightly press the region near the borders of the fingernails of the fourth finger (medical finger) L4 and the fifth finger (little finger) L5 of the left hand or the regions near the borders of the fingernails of the second finger (forefinger) R2 and the third finger (long finger) R3 of the right hand.

If a power switch 182 (not shown) on an operation panel 128 (not shown) is turned on in this state, then a sequence/timer circuit 183 (not shown) starts and carries current across the near-infrared LEDs 141 to 146 and blue-violet LEDs 171 to 176 according to set conditions.

Among the LEDs, the near-infrared LEDs 141 to 146 are repeatedly turned on or off in a predetermined period during timer set time by functions of a pulse generation circuit 184 (not shown) and a switching circuit 185 (not shown). As a result, the near-infrared rays are intermittently irradiated on the regions near the borders of the fingernails of the five fingers L1 to L5 of the left hand inserted into the respective fingertip insertion units 131 to 135 or those of the five fingers R1 to R5 of the right hand inserted into the respective fingertip insertion units 136 to 132.

On the other hand, the blue-violet LEDs 171 to 176 continue to be turned on during timer set time. As a result, blue-violet lights are continuously irradiated onto the fingertip ball or pad-side regions of the five fingers L1 to L5 of the left hand inserted into the respective fingertip insertion units 131 to 135 or those of the five fingers R1 to R5 of the right hand inserted into the respective fingertip insertion units 136 to 132.

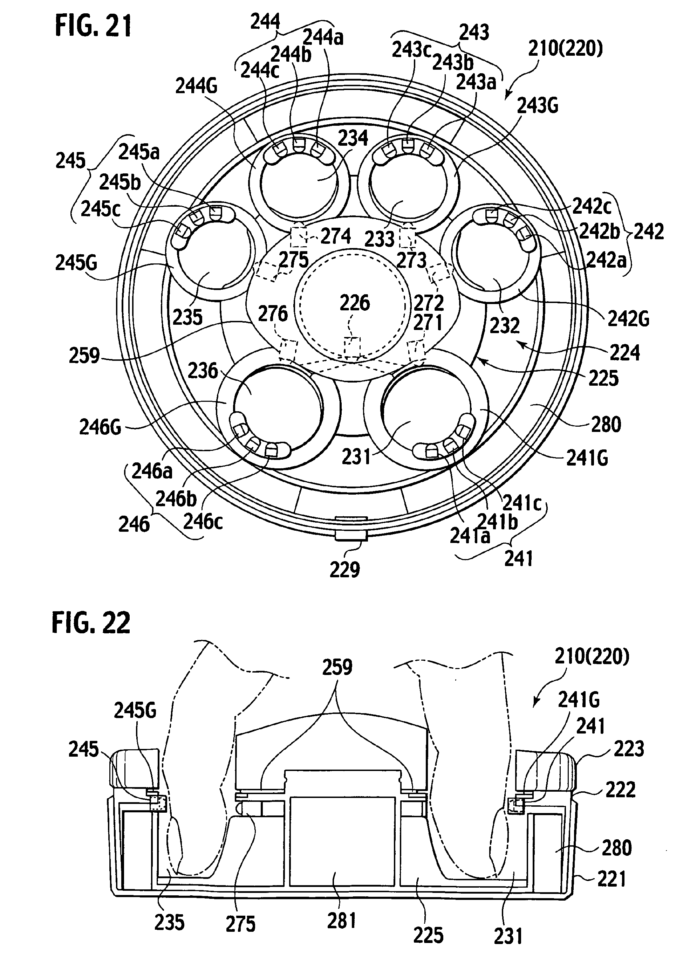

Figs. 21 and 22 show a fourth embodiment of the fingertip stimulating apparatus according to the present invention. Fig. 21 is a cross-sectional plan view (corresponding to Fig. 8) of principal parts, and Fig. 22 is a longitudinal side view (corresponding to Fig. 9) of the principal parts.

A fingertip stimulating apparatus 210 shown therein is almost similar to the fingertip stimulating apparatus 10 shown in Figs. 4 to 9 (and Figs. 10 to 18). Therefore, like constituent elements are denoted by reference numerals that are like reference numerals plus 200 as those used in the fingertip stimulating apparatus 10, and detailed descriptions and illustrations thereof will be omitted.

The fingertip stimulating apparatus 210 is configured so that near-infrared LEDs 241a, 241b, and 241c of a fingertip insertion unit 231 are fixed to one ring attached to a flexible band to constitute an LED-band-added ring 241G. The LED-band-added ring 241G is arranged so that its position can be adjusted in the fingertip insertion unit 231 by being slightly moved when a fingertip is inserted into the fingertip insertion unit 231.

Further, the fingertip stimulating apparatus 210 is configured so that near-infrared LEDs 242a, 242b, and 242c of a fingertip insertion unit 232 are fixed to one ring attached to a flexible band to constitute an LED-band-added ring 242G. The LED-band-added ring 242G is arranged so that its position can be adjusted in the fingertip insertion unit 232 by being slightly moved when a fingertip is inserted into the fingertip insertion unit 232.

Moreover, the fingertip stimulating apparatus 210 is configured so that near-infrared LEDs 243a, 243b, and 243c of a fingertip insertion unit 233 are fixed to one ring attached to a flexible band to constitute an LED-band-added ring 243G. The LED-band-added ring 243G is arranged so that its position can be adjusted in the fingertip insertion unit 233 by being slightly moved when a fingertip is inserted into the fingertip insertion unit 233.

Furthermore, the fingertip stimulating apparatus 210 is configured so that near-infrared LEDs 244a, 244b, and 244c of a fingertip insertion unit 234 are fixed to one ring attached to a flexible band to constitute an LED-band-added ring 244G. The LED-band-added ring 244G is arranged so that its position can be adjusted in the fingertip insertion unit 234 by being slightly moved when a fingertip is inserted into the fingertip insertion unit 234.

Further, the fingertip stimulating apparatus 210 is configured so that near-infrared LEDs 245a, 245b, and 245c of a fingertip insertion unit 235 are fixed to one ring attached to a flexible band to constitute an LED-band-added ring 245G. The LED-band-added ring 245G is arranged so that its position can be adjusted in the fingertip insertion unit 235 by being slightly moved when a fingertip is inserted into the fingertip insertion unit 235.

Moreover, the fingertip stimulating apparatus 210 is configured so that near-infrared LEDs 246a, 246b, and 246c of a fingertip insertion unit 236 are fixed to one ring attached to a flexible band to constitute an LED-band-added ring 246G. The LED-band-added ring 246G is arranged so that its position can be adjusted in the fingertip insertion unit 236 by being slightly moved when a fingertip is inserted into the fingertip insertion unit 236.

A retaining press plate 259 presses upper surfaces of the LED-band-added rings 241G to 246G so as to prevent the LED-band-added rings 241G to 246G from being coming out upward when the inserted fingers are pulled out from the respective LED-band-added rings 241G to 246G.

When the above-stated fingertip stimulating apparatus 210 is used, the first finger (thumb) L1, the second finger (forefinger) L2, the third finger (long finger) L3, the fourth finger (medical finger) L4, and the fifth finger (little finger) L5 of the left hand are inserted into respective fingertip insertion units 231, 232, 233, 234, and 235 or the first finger (thumb) R1, the second finger (forefinger) R2, the third finger (long finger) R3, the fourth finger (medical finger) R4, and the fifth finger (little finger) R5 of the right hand are inserted into respective fingertip insertion units 236, 235, 234, 233, and 232.

The LED-band-added ring 241G is arranged at the finger insertion unit 231 so that the position of the LED-band-added ring 241G can be adjusted during insertion of the fingertip. Due to this, by inserting the fingertip, the near-infrared LEDs 241a, 241b, and 241c attached to the LED-band-added ring 241G nicely touch a region near a border of a fingernail of the first finger (thumb) L1 of the left hand.

The LED-band-added ring 242G is arranged at the finger insertion unit 232 so that the position of the LED-band-added ring 242G can be adjusted during insertion of the fingertip. Due to this, by inserting the fingertip, the near-infrared LEDs 242a, 242b, and 242c attached to the LED-band-added ring 242G nicely touch a region near a border of a fingernail of the second finger (forefinger) L2 of the left hand or of the fifth finger (little finger) R5 of the right hand.

The LED-band-added ring 243G is arranged at the finger insertion unit 233 so that the position of the LED-band-added ring 243G can be adjusted during insertion of the fingertip. Due to this, by inserting the fingertip, the near-infrared LEDs 243a, 243b, and 243c attached to the LED-band-added ring 243G nicely touch a region near a border of a fingernail of the third finger (long finger) L3 of the left hand or of the fourth finger (medical finger) R4 of the right hand.

The LED-band-added ring 244G is arranged at the finger insertion unit 234 so that the position of the LED-band-added ring 244G can be adjusted during insertion of the fingertip. Due to this, by inserting the fingertip, the near-infrared LEDs 244a, 244b, and 244c attached to the LED-band-added ring 244G nicely touch a region near a border of a fingernail of the fourth finger (medical finger) L4 of the left hand or of the third finger (long finger) R3 of the right hand.

The LED-band-added ring 245G is arranged at the finger insertion unit 235 so that the position of the LED-band-added ring 245G can be adjusted during insertion of the fingertip. Due to this, by inserting the fingertip, the near-infrared LEDs 245a, 245b, and 245c attached to the LED-band-added ring 245G nicely touch a region near a border of a fingernail of the fifth finger (little finger) L5 of the left hand or of the second finger (forefinger) R2 of the right hand.

The LED-band-added ring 246G is arranged at the finger insertion unit 236 so that the position of the LED-band-added ring 246G can be adjusted during insertion of the fingertip. Due to this, by inserting the fingertip, the near-infrared LEDs 246a, 246b, and 246c attached to the LED-band-added ring 246G nicely touch a region near a border of a fingernail of the first finger (thumb) R1 of the right hand.

If a power switch 282 (not shown) on an operation panel 228 (not shown) is turned on in this state, then a sequence/timer circuit 283 (not shown) starts and carries current across the near-infrared LEDs 241 to 246 and blue-violet LEDs 271 to 276 according to set conditions.

Among the LEDs , the near-infrared LEDs 241 to 246 are repeatedly turned on or off in a predetermined period during timer set time by functions of a pulse generation circuit 284 (not shown) and a switching circuit 285 (not shown). As a result, the near-infrared rays are intermittently irradiated on the regions near the borders of the fingernails of the five fingers L1 to L5 of the left hand inserted into the respective fingertip insertion units 231 to 235 or those of the five fingers R1 to R5 of the right hand inserted into the respective fingertip insertion units 236 to 232.

On the other hand, the blue-violet LEDs 271 to 276 continue to be turned on during timer set time. As a result, blue-violet lights are continuously irradiated onto the fingertip ball or pad-side regions of the five fingers L1 to L5 of the left hand inserted into the respective fingertip insertion units 231 to 235 or those of the five fingers R1 to R5 of the right hand inserted into the respective fingertip insertion units 236 to 232.

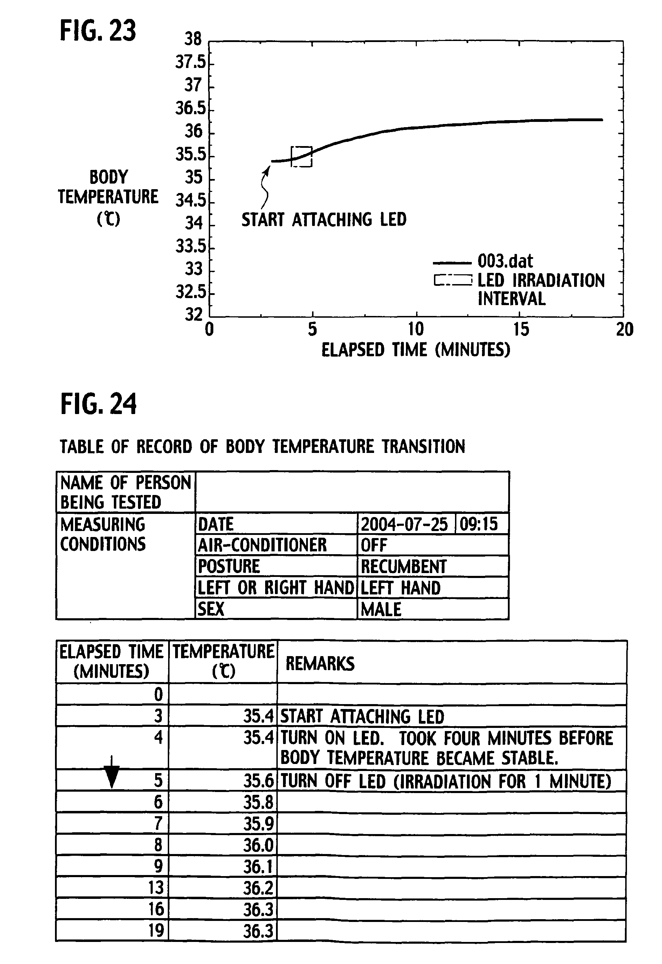

Fig. 23 is a graph showing a first experiment on transition of the body temperature of a person being tested obtained by irradiation of the near-infrared ray using the fingertip stimulating apparatus 10. Fig. 24 shows a record of the body temperature transition.

As evident from Figs. 23 and 24, by irradiating the near-infrared ray onto the regions of the borders of the fingernails of the fingertips for a short time using the fingertip stimulating apparatus 10, the body temperature of the person being tested rose and the more suitable body temperature than an initial body temperature was maintained subsequently for a long time.

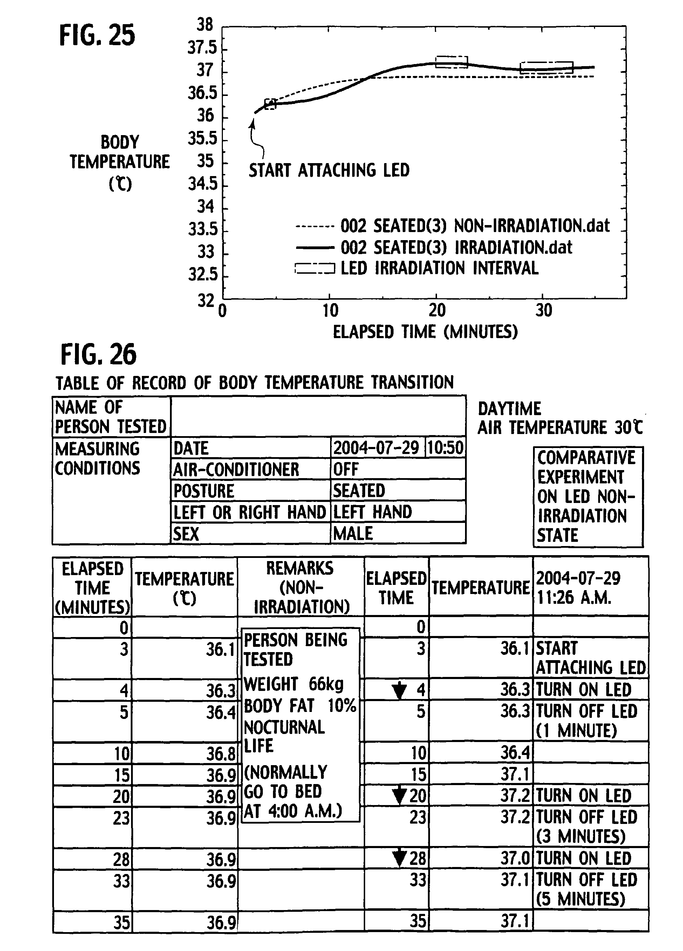

Fig. 25 is a graph showing a second experiment on transition of the body temperature of a person being tested obtained by irradiation of the near-infrared ray using the fingertip stimulating apparatus 10. Fig. 26 shows a record of the body temperature transition.

As evident from Figs. 25 and 26, by irradiating the near-infrared ray onto the regions of the borders of the fingernails of the fingertips for a short time using the fingertip stimulating apparatus 10, the body temperature of the person being tested rose. When the near-infrared ray is subsequently irradiated for a short time, the stable body temperature was maintained.

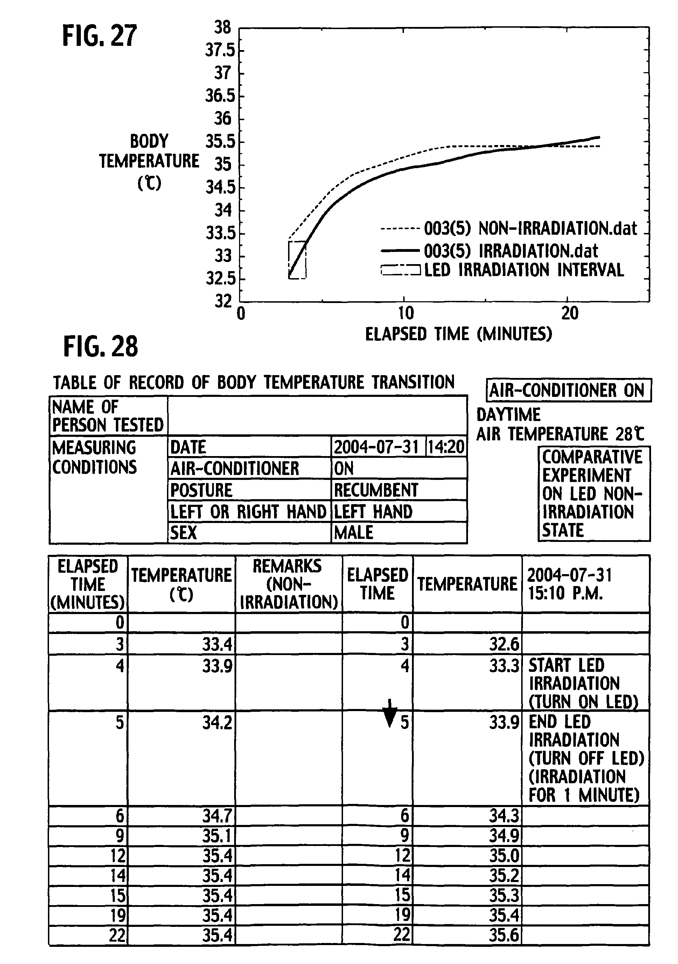

Fig. 27 is a graph showing a third experiment on transition of the body temperature of a person being tested obtained by irradiation of the near-infrared ray using the fingertip stimulating apparatus 10. Fig. 28 shows a record of the body temperature transition.

As evident from Figs. 27 and 28, by irradiating the near-infrared ray onto the regions of the borders of the fingernails of the fingertips for a short time using the fingertip stimulating apparatus 10, the body temperature of the person being tested with quite a low initial body temperature quickly rose and the body temperature subsequently continued to rise toward a normal value.

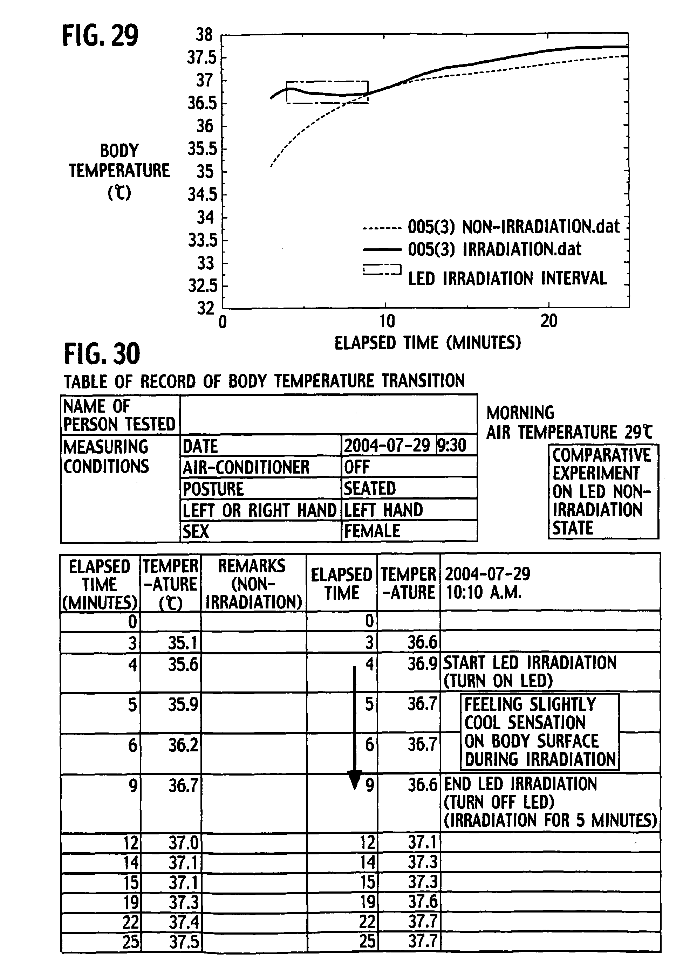

Fig. 29 is a graph showing a fourth experiment on transition of the body temperature of a person being tested obtained by irradiation of the near-infrared ray using the fingertip stimulating apparatus 10. Fig. 30 shows a record of the body temperature transition.

As evident from Figs. 29 and 30, by irradiating the near-infrared ray onto the regions of the borders of the fingernails of the fingertips for a short time using the fingertip stimulating apparatus 10, the body temperature of the person being tested rose and was gradually stabilized subsequently.

It is understood from results of the above-stated experiments that it is possible to ensure stimulating the fingertips by irradiating the near-infrared ray onto the regions near the borders of the fingernails of the fingertips using the fingertip stimulating apparatus 10. Furthermore, this fingertip stimulation can provide a safe and effective stimulus for the autonomic nerves and enables body temperature control.

It is also understood that, even if the other fingertip stimulating apparatus 1, 110 or 210 than the fingertip stimulating apparatus 10 is used, the similar fingertip stimulation can provide a safe and effective stimulus for the autonomic nerves and enables body temperature control.

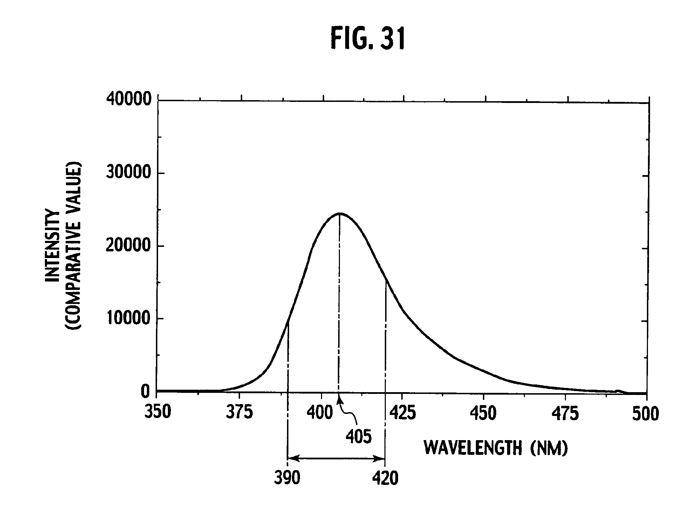

Fig. 31 is a graph showing an intensity distribution of an emission spectrum of a fluorescent lamp in a band of 400 nm developed by the present inventors. In the graph, a horizontal axis indicates wavelength and a vertical axis indicates a value proportional to an enumerated value (photon number) by a photomultiplier tube of a spectrometer.

As evident from Fig. 31, the fluorescent lamp characteristically has a peak center wavelength of 405 nm and a half bandwidth of 33.0 nm.

The present inventors conducted an experiment using the fluorescent lamp and accidentally discovered the effect of improving an oxygen saturation in the blood by irradiating a light in a band of 400 nm on a person.

It is known that if the oxygen saturation in the blood lowers, various diseases such as a breathing disorder and cyanosis concur in a living body. Therefore, it is considered that the effect of improving the oxygen saturation in the blood by irradiation of the light in the band of 400 nm is favorable for a human body.

The purpose of the blue-violet LEDs 71 to 76 provided in the fingertip stimulating apparatus 10 is to attain the effect of improving the oxygen saturation in the blood by irradiation of the light in the band of 400 nm onto the human body similarly to the fluorescent lamp in the band of 400 nm.

The effect is realized by selecting, as the blue-violet LEDs 71 to 76, LEDs at wavelengths preferably in the range of 390 nm to 420 nm, more preferably near 405 nm. This is because emission spectrums of the blue-violet LEDs 71 to 76 substantially overlap with that of the fluorescent lamp in the band of 400 nm.

In this case, purposes of the blue-violet light generating member 7, the blue-violet LEDs 171 to 176, and the blue-violet LEDs 271 to 276 provided in the other fingertip stimulating apparatuses 1, 110 and 210 than the fingertip stimulating apparatus 10, respectively, are to attain the effect of improving the oxygen saturation in the blood by irradiation of the light in the band of 400 nm onto the human body similarly to the fluorescent lamp in the band of 400 nm.

In the embodiments, all the fingertip stimulating apparatuses 1, 10, 110, and 210 include the near-infrared generating member 4, the near-infrared LEDs 41 to 46, 141 to 146, and 241 to 246, the blue-violet light generating member 7, and the blue-violet LEDs 71 to 76, 171 to 176, and 271 to 276. However, the present invention is not limited to the embodiments.

Namely, the fingertip stimulating apparatuses 1, 10, 110, and 210 can be configured by providing only the near-infrared generating member 4 and the near-infrared LEDs 41 to 46, 141 to 146, and 241 to 246 without providing the blue-violet light generating member 7, and the blue-violet LEDs 71 to 76, 171 to 176, and 271 to 276.

Alternatively, the fingertip stimulating apparatuses 1, 10, 110, and 210 can be configured by providing only the blue-violet light generating member 7, and the blue-violet LEDs 71 to 76, 171 to 176, and 271 to 276 without providing the near-infrared generating member 4 and the near-infrared LEDs 41 to 46, 141 to 146, and 241 to 246.

Moreover, in the embodiments, the band pressure cams 53, 63a, and 63b and the cam drive rods 56, 66a, and 66b or the band forcing members 157, 167a, and 167b including the compression springs 158, 168a, and 168b are used so as to cause the near-infrared LEDs 41 to 46 and 141 to 146 to touch and lightly press the regions near the fingernails of the respective fingertips, as illustrated in the fingertip stimulating apparatuses 10 and 110. However, the present invention is not limited to the embodiments.

Namely, it is possible to cause the near-infrared LEDs 41 to 46 and 141 to 146 to touch and lightly press the regions near the fingernails of the respective fingertips by the function of the finer pad members 25 and 125 for pushing out the ball or pad sides of the fingertips by expanding the finger pad members 25 and 125 outward of the radial direction of the apparatus main bodies 20 and 120 using, for example, air pressure.

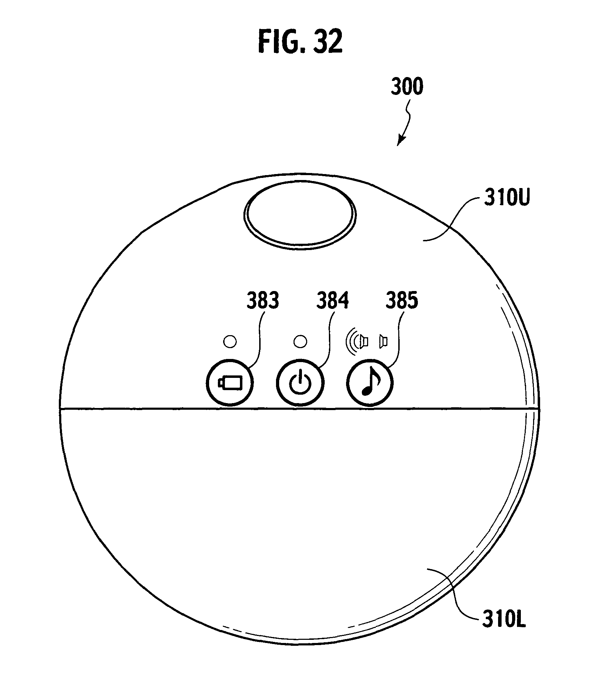

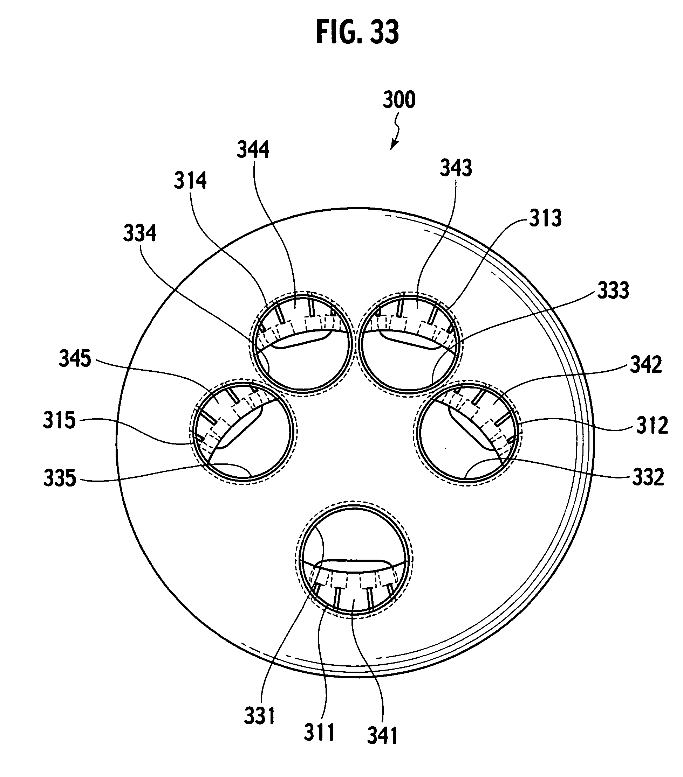

Figs. 32 to 49 show a fifth embodiment of a fingertip stimulating apparatus according to the present invention.

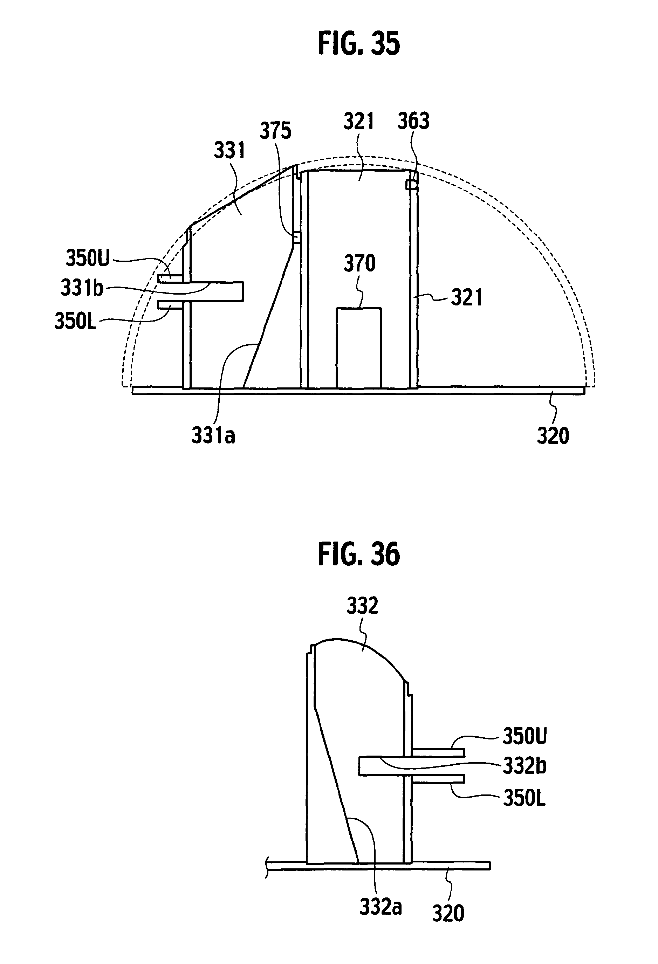

As shown in Fig. 5, a fingertip stimulating apparatus 300 is constituted substantially spherically by an upper case 310U and a lower case 310L that are semispherical and detachable from each other. As shown in Fig. 35, a support plate 320 having a circular outline is arranged near a boundary between the upper case 310U and the lower case 310L.

A hollow cylinder 321 is attached to a center of the support plate 320 and formed to have such a length that an upper end of the hollow cylinder 321 extends to abut the upper case 310U. The hollow cylinder 321 is made of light transmission plastic.

Furthermore, five fingertip insertion units 331 to 335 circumscribed with the hollow cylinder 321. The fingertip insertion units 331 to 335 are substantially cylindrical, formed to have such a length that upper ends of the fingertip insertion units 331 to 335 extend to abut the upper case 310U, and attached to five openings 311 to 315 formed in the upper case 310U, respectively. Each of the fingertip insertion units 331 to 335 is made of light transmission plastic.

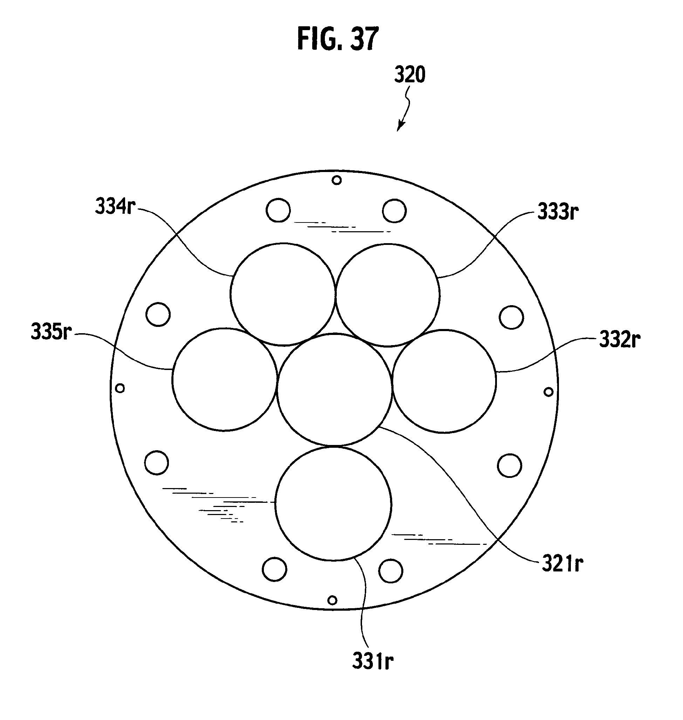

As shown in Fig. 37, a concave portion 321r for positioning a lower end of the hollow cylinder 321 and concave portions 331r, 332r, 333r, 334r, and 335r for positioning lower ends of the respective fingertip insertion units 331 to 335 are formed on an upper surface of the support plate 320. This can facilitate positioning the hollow cylinder 321 and the fingertip insertion units 331 to 335.

One fingertip insertion unit 331 is a unit into which a fingertip of a thumb is inserted and formed into a circular shape larger in diameter than the other four fingertip insertion units 332, 333, 334, and 335. The other four fingertip insertion units 332, 333, 334, and 335 are units into which fingertips of four fingers other than the thumb are inserted, respectively, and formed into cylindrical shapes equal in diameter so as to be shared between the right and left hands.

Inclined guide units 331a, 332a, 333a, 3334a, and 335a for guiding the inserted fingers in certain directions are formed in lower portions of the fingertip insertion units 331, 332, 333, 334, and 335, respectively.

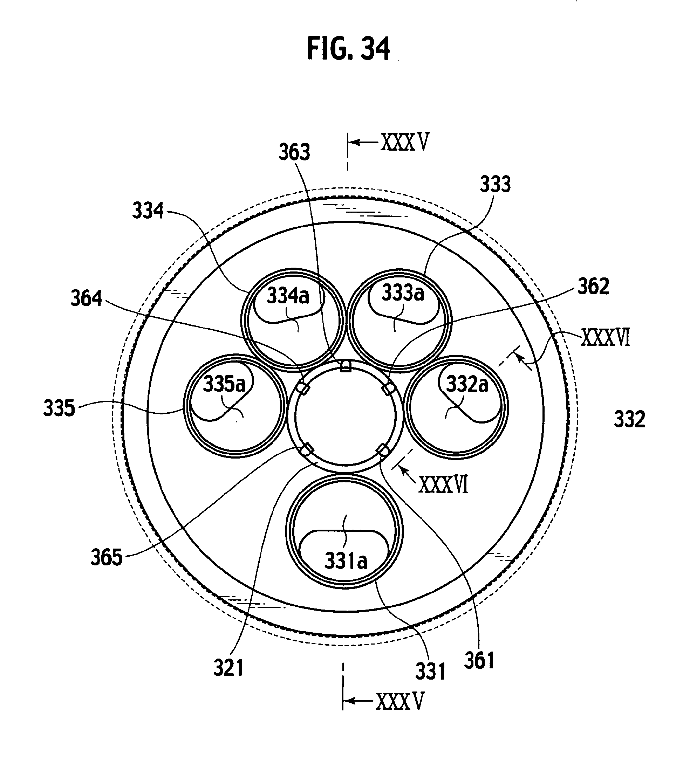

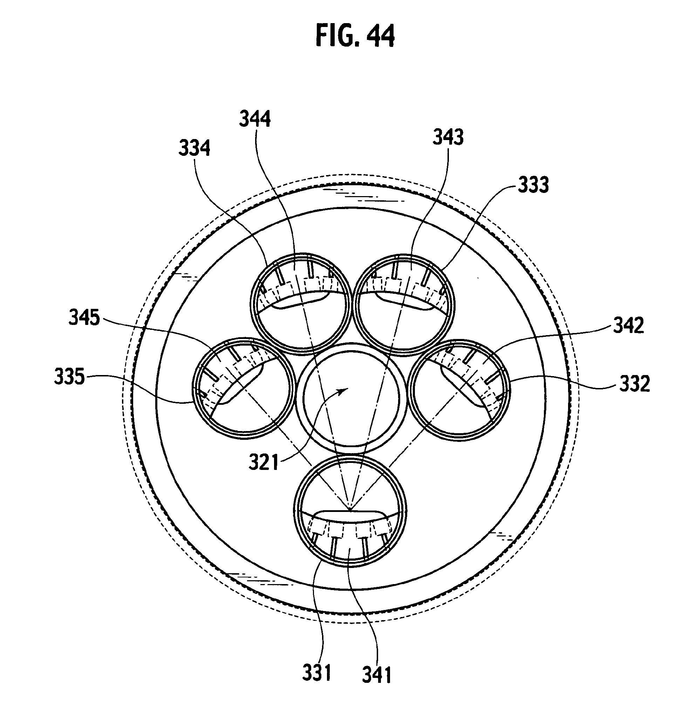

As shown in Figs. 34 and 44, the guide unit 331a for the fingertip insertion unit 331 guides the fingertip in an extension direction of a line extending from a center of the hollow cylinder 321 to a center of the thumb fingertip insertion unit 331.

Furthermore, the guide units 332a, 333a, 334a, and 335a for the fingertip insertion units 332, 333, 334, and 335 for the fingers other than the thumb guide the fingertips in extension directions of lines extending from the center of the thumb fingertip insertion unit 331 to centers of the fingertip insertion units 332, 333, 334, and 335, respectively.

As shown in Fig. 35, a window hole 331b having a length substantially half a circumference of the fingertip insertion unit 331 is formed at a predetermined height on a side opposed to the guide unit 331a.

As shown in Fig. 36, a window hole 332b having a length substantially half a circumference of the fingertip insertion unit 332 is formed at a predetermined height on a side opposed to the guide unit 332a. Likewise, although not shown in the drawings, window holes 333b, 334b, and 335b (not shown) having length substantially half circumferences of the fingertip insertion unit 333, 334, and 335 are formed at a predetermined height on a side opposed to the guide units 333a, 334a, and 335a, respectively.

These window holes 331b, 332b, 333b, 334b, and 335b are all arranged at the same height. The height is designed to a height at which borders of fingernails of fingers are located when the fingers are inserted into the respective fingertip insertion units 331, 332, 333, 334, and 335.

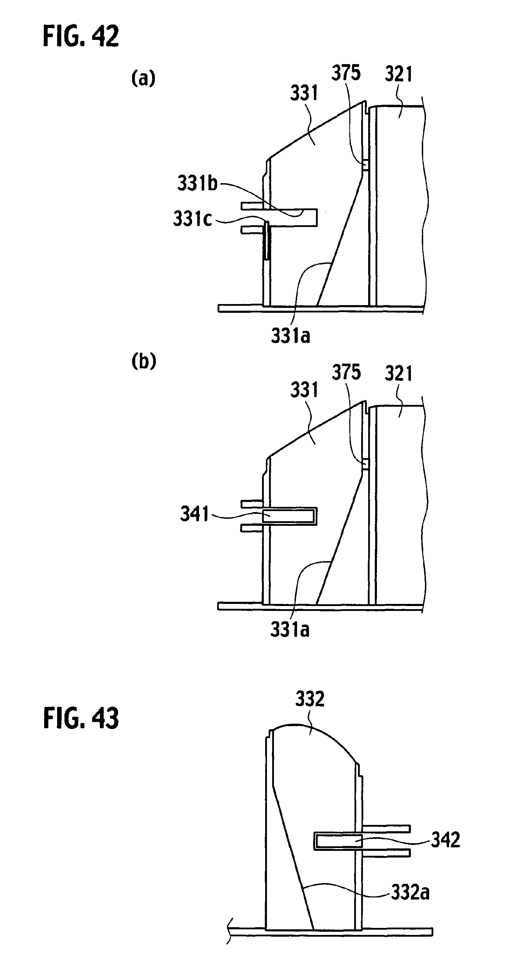

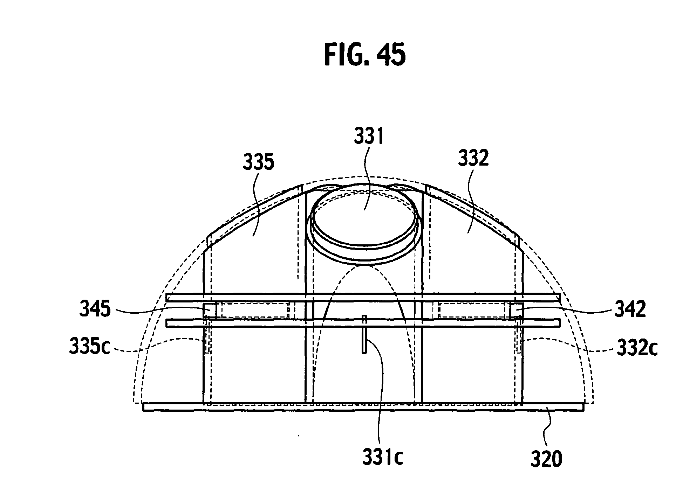

As shown in Figs. 42 (a) and 45 (b), the window hole 331b of the fingertip insertion unit 331 for the thumb includes a pin 331c on a line extending from the center of the hollow cylinder 321 to the center of the thumb fingertip insertion unit 331. The window hole 331b is formed to have identical lengths on its both sides across the pin 331c, respectively.

As shown in Fig. 45, the window holes 332b and 335b of the fingertip insertion units 332 and 335 include pins 332c and 335c on lines extending from the center of the thumb insertion unit 331 to the centers of the fingertip insertion unit 332 and 335 (see Fig. 44), respectively. In Fig. 45, the pins 332c and 335c are shown to be deviated from their original positions. The window holes 332b and 335b are formed to have identical lengths on their both sides across the pins 332c and 335c, respectively.

Likewise, although not shown in the drawings, the window holes 333b and 334b of the fingertip insertion units 333 and 334 include pins 333c and 334c (not shown) on lines extending from the center of the thumb insertion unit 331 to the centers of the fingertip insertion unit 333 and 334 (see Fig. 44), respectively. The window holes 333b and 334b are formed to have identical lengths on their both sides across the pins 333c and 334c, respectively.

As shown in Fig. 42 (b), an LED unit 341 having a height at which the LED unit 341 is insertable with a required clearance to a height of the window hole 331b of the thumb insertion unit 331 (a distance between an upper portion and a lower portion of the window hole 331b).

As shown in Fig. 43 , an LED unit 342 having a height at which the LED unit 342 is insertable with a required clearance to a height of the window hole 332b of the insertion unit 332 (a distance between an upper portion and a lower portion of the window hole 332b).

Likewise, LED units 343, 344, and 345 having heights at which the LED 343, 344, and 345 are insertable with required clearances to heights of the window holes 333b, 334b, and 335b of the insertion units 333, 334, and 335 (distances between upper portions and lower portions of the respective window holes 333b, 334b, and 335b).

Since the window holes 331b, 332b, 333b, 334b, and 335b are equal in height (distance between the upper and lower portions), the LED units 341, 342, 343, 344, and 345 are all equal in height.

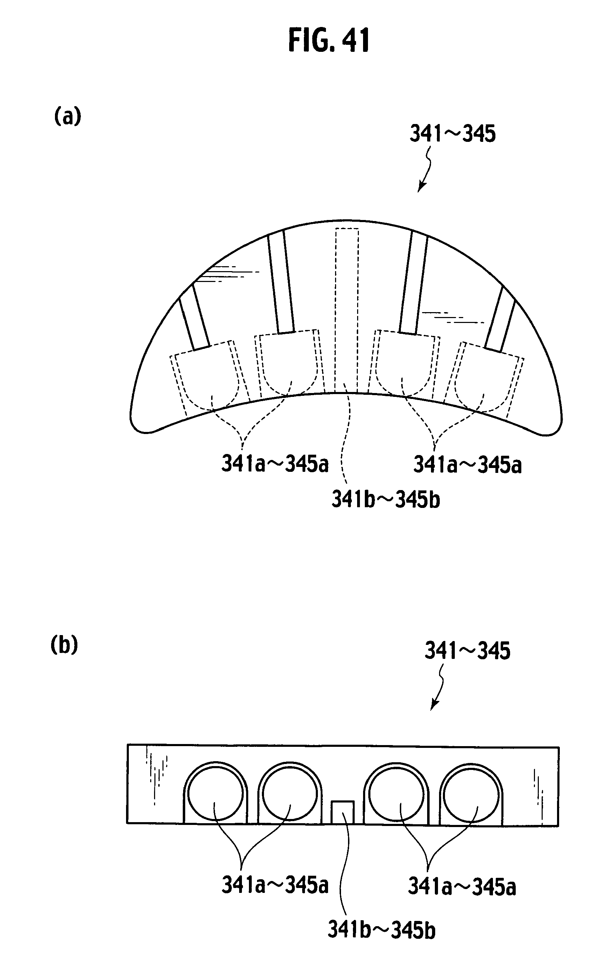

As shown in Fig. 41, all of the LED units 341, 342, 343, 344, and 345 are substantially crescent-shaped. The LED units 342, 343, 344 and 345 are identical while the LED unit 341 is slightly larger than the LED units 342, 343, 344 and 345.

Namely, a length of the LED unit 341 is substantial equal to an outside diameter of the fingertip insertion unit 331, a back surface of the LED unit 341 is substantially equal in curvature to the outline of the fingertip insertion unit 331, and four near-infrared LEDs 341a are arranged on a front surface of the LED unit 341 larger in curvature than the back surface in a direction of the center of curvature of the front surface.

Further, lengths of the LED units 342, 343, 344, and 345 are substantial equal to outside diameters of the fingertip insertion units 332, 333, 334, and 335, back surfaces of the LED units 342, 343, 344, and 345 are substantially equal in curvature to outlines of the fingertip insertion units 332, 333, 334 and 335, and four near-infrared LEDs 342a, 343a, 344a, and 345a are arranged on front surfaces of the LED units 342, 343, 344, and 345 higher in curvature than the back surfaces in directions of centers of curvature of the front surfaces, respectively.

A wavelength of a near-infrared ray emitted from each of the near-infrared LEDs 341a, 342a, 343a, 344a, and 345a is preferably in a range of 820 nm to 920 nm, more preferably near 870 nm. It is preferable to intermittently turn on or off the near-infrared LEDs 341a, 342a, 343a, 344a, and 345a so as to irradiate near-infrared rays onto regions near borders of the fingernails of the fingertips and to stimulate the fingertips, respectively.

The LED units 341, 342, 343, 344, and 345 include long grooves 341b, 342b, 343b, 344b, and 345b along central axes between left and right sides thereof at the center of bottoms of the respective LED units 341, 342, 343, 344, and 345. The long grooves 341b, 342b, 343b, 344b, and 345b are engaged with the pins 331c, 332c, 333c, 334c, and 335c. By doing so, the near-infrared LEDs 341a, 342a, 343a, 344a, and 345a arranged on the front surfaces of the LED units 341, 342, 343, 344, and 345 can enter the fingertip insertion units 331, 332, 333, 334, and 335 from the window holes 331b, 332b, 333b, 334b, and 335b, respectively.

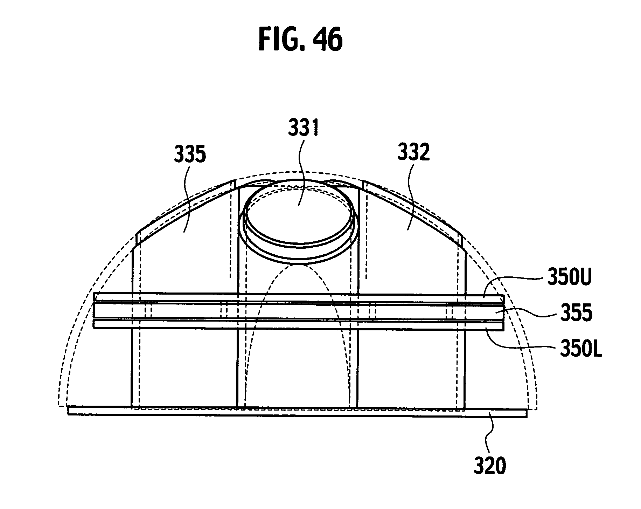

As shown in Fig. 46, each of the LED units 341, 342, 343, 344, and 345 is held at a predetermined position by an upper support plate 350U and a lower support plate 350L between which an appropriate cushion member 355 is put.

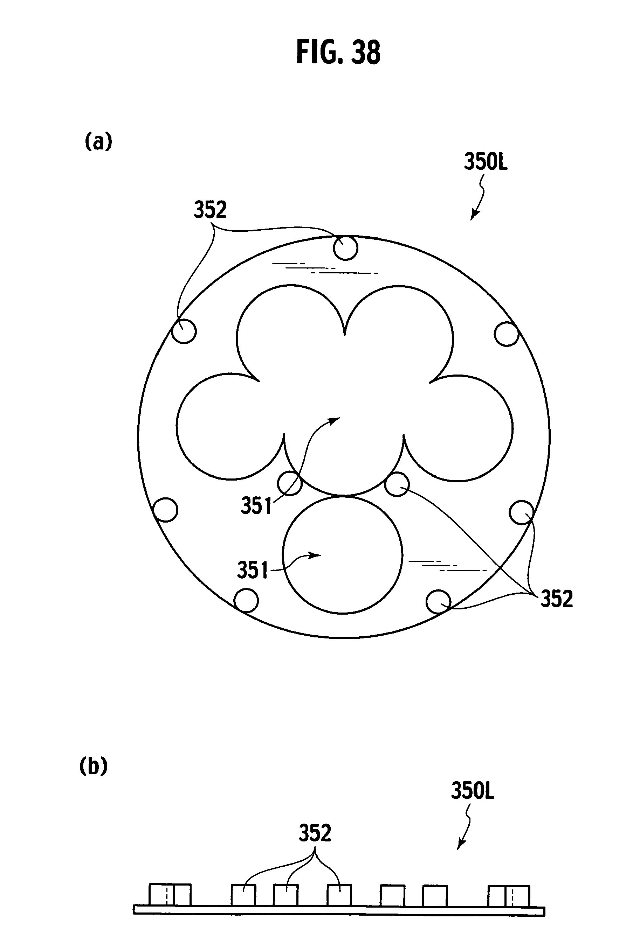

As shown in Fig. 38, the lower support plate 350L is of a shape formed by cutting off arrangement regions 351 in which the hollow cylinder 321 and the five fingertip insertion units 331, 332, 333, 334, and 335 are arranged from a circular plate member. The lower support plate 350L includes a plurality of dowels 352 on its upper surface.

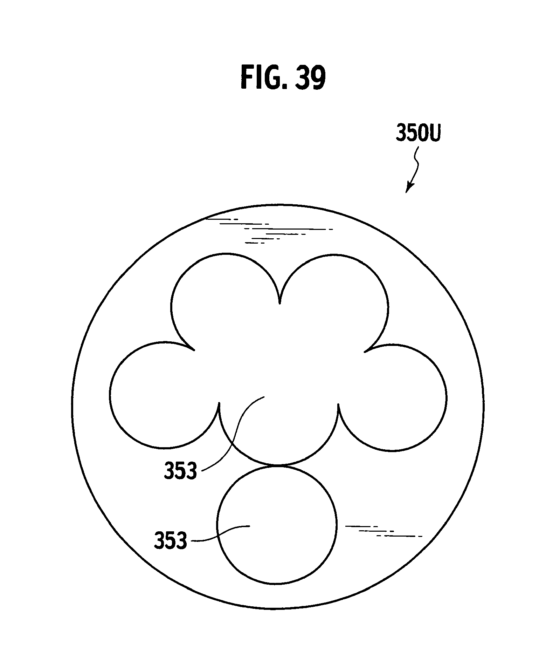

As shown in Fig. 39, the upper support plate 350U is of a shape formed by cutting off arrangement regions 353 in which the hollow cylinder 321 and the five fingertip insertion units 331, 332, 333, 334, and 335 are arranged from a circular plate member.

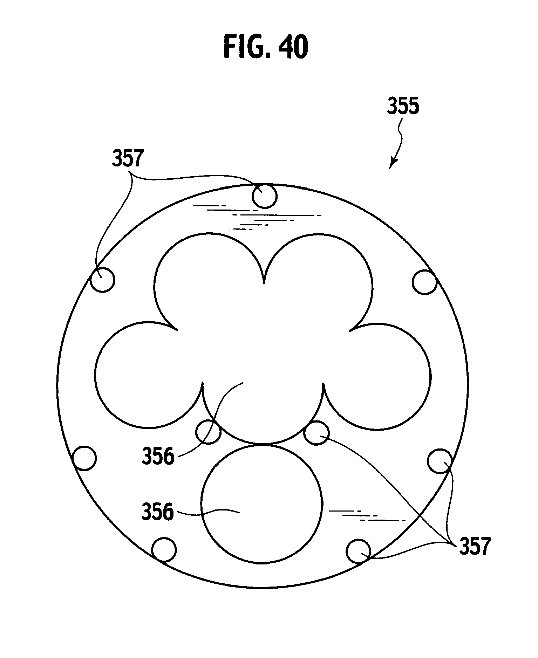

As shown in Fig. 40, the cushion member 355 is of a shape formed by cutting of arrangement regions 356 in which the hollow cylinder 321 and the five fingertip insertion units 331, 332, 333, 334, and 335 are arranged from a circular cushion member having a thickness substantially equal to the height of each of the window holes 331b, 332b, 333b, 334b, and 335b (distance between the upper and lower portion of each of the window holes 331b, 332b, 333b, 334b, and 335b). The cushion member 355 includes a plurality of holes 357 corresponding to the dowels 352 of the lower support plate 350L.

As such a cushion member 355, a cushion member made of, for example, low bounce urethane.

As shown in Figs. 35 and 36, the lower support plate 350L is attached to a position at a height protruding from a lower surface of each of the window holes 331b, 332b, 333b, 334b, and 335b. The upper support plate 350U is attached to a position at a height protruding from an upper surface of each of the window holes 331b, 332b, 333b, 334b, and 335b.

Furthermore, the cushion member 355 is put between the upper and lower support plates 350U and 350L in a sandwich fashion as shown in Fig. 46 by engaging the holes 357 with the corresponding dowels 352 of the lower support plate 350L, respectively.

The cushion member 355 enables each of the LED units 341, 342, 343, 344, and 345 to be held at such a position that the back surface overlaps with the outline of each of the fingertip insertion units 331, 332, 333, 334, and 335.

Namely, the cushion member 355 is closely attached to back surfaces of the five LED units 341, 342, 343, 344, and 345 that enter the fingertip insertion units 331, 332, 333, 334, and 335 up to entrance limits in an initial state where no external force is applied. Further, the cushion member 355 functions to be able to be greatly compressed or deformed according to the external force when the external force is applied, and to be able to be restored to its original shape when the external force is eliminated.

Because of the function of the cushion member 355, when the fingertips are inserted into the fingertip insertion units 331, 332, 333, 334, and 335, the LED units 341, 342, 343, 344, and 345 are pressed by the inserted fingers, whereby the LED units 341, 342, 343, 344, and 345 slide rearward so as not to prevent entry of the fingertips while compressing and deforming the cushion member 355.

At this time, the LED units 341, 342, 343, 344, and 345 can oscillate around the pins 331c, 332c, 333c, 334c, and 335c, respectively. Therefore, a combined movement of the oscillation movement and the rearward sliding movement can facilitate positioning the near-infrared LEDs 341a, 342a, 343a, 344a, and 345a to the borders of the fingernails of the respective fingertips.

Due to this, when the near-infrared LEDs 341a, 342a, 343a, 344a, and 345a are activated, near-infrared rays are irradiated onto the borders of the fingernails of the respective fingertips of the user.

When the fingertips are pulled out from the respective fingertip insertion units 331, 332, 333, 334, and 335, the LED units 341, 342, 343, 344, and 345 automatically slide forward and return to initial positions by the function of the cushion member 355 restored from the compressed and deformed state to the initial state.

As shown in Figs. 34 and 44, the fingertip stimulating apparatus 300 also includes five blue-violet LEDs 361, 362, 363, 364, and 365 near an upper end of the hollow cylinder 321.

A wavelength of a blue-violet light emitted from each of the blue-violet LEDs 361, 362, 363, 364, and 365 is preferably in a range of 390 nm to 420 nm, more preferably near 405 nm.

It is also preferable to intermittently turn on or off the blue-violet LEDs 361, 362, 363, 364, and 365 so as to stimulate visual sensation of the user.

As shown in Figs. 34 and 35, the blue-violet LEDs 361, 362, 363, 364, and 365 are arranged to have the same height at intermediate positions between adjacent fingertip insertion units 331, 332, 333, 334, and 335, respectively, along a circumferential direction of the hollow cylinder 321. The blue-violet LEDs 361, 362, 363, 364, and 365 emit lights outward at the respective positions.

Due to this, when the blue-violet LEDs 361, 362, 363, 364, and 365 are activated, the blue-violet lights are diffused upward from the openings 311, 312, 313, 314, and 315 of the upper case 310U to which upper ends of the fingertip insertion units 331, 332, 333, 334, and 335 are attached, respectively, and visually recognized by the user.

As shown in Fig. 35, the fingertip stimulating apparatus 300 includes a vibration unit 370 arranged within the hollow cylinder 321 and fixed to the support plate 320. The vibration unit 370 is configured so that two vibrators 370a and 370b (not shown) substantially disk-shaped and producing vibrations by being rotated about their central axes are arranged with the central axes orthogonal to each other. Due to this, when the vibration unit 370 operates, a whizzing sound is produced.

As shown in Figs. 34 and 42, a photosensor 375 that detects insertion of a fingertip is provided in an arbitrary one of the fingertip insertion units 331 to 335, e.g., the thumb fingertip insertion unit 331. When the user inserts his or her fingertips into the respective fingertip insertion units 331 to 335, the photosensor 375 detects insertion of the fingertip. The detection is used as a start condition for a preset operation sequence in one cycle of the fingertip stimulating apparatus 300.

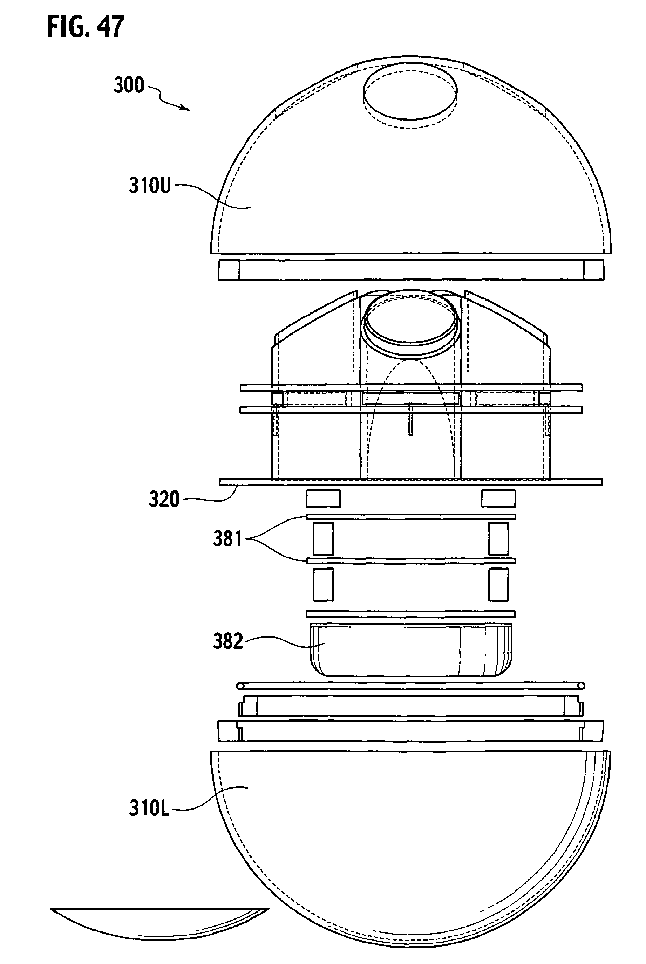

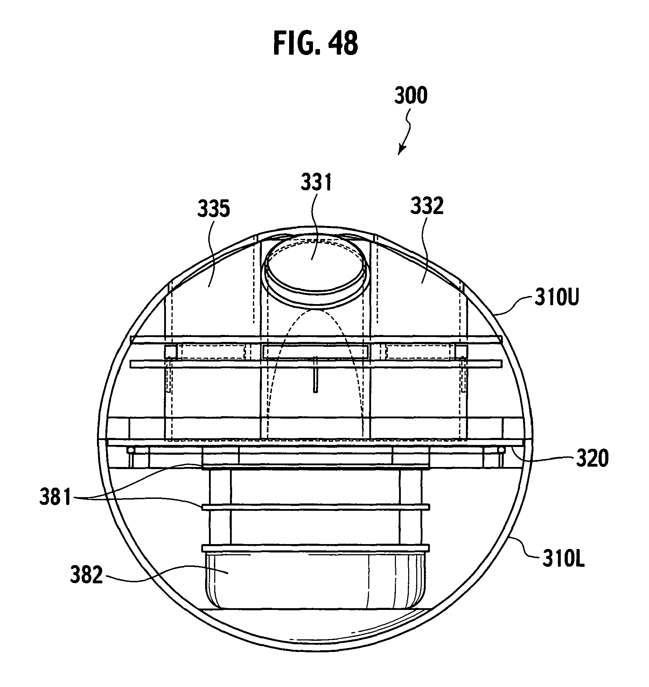

As shown in Figs. 47 and 48, printed boards 381, a battery 382 and the like are provided below the support plate 320.

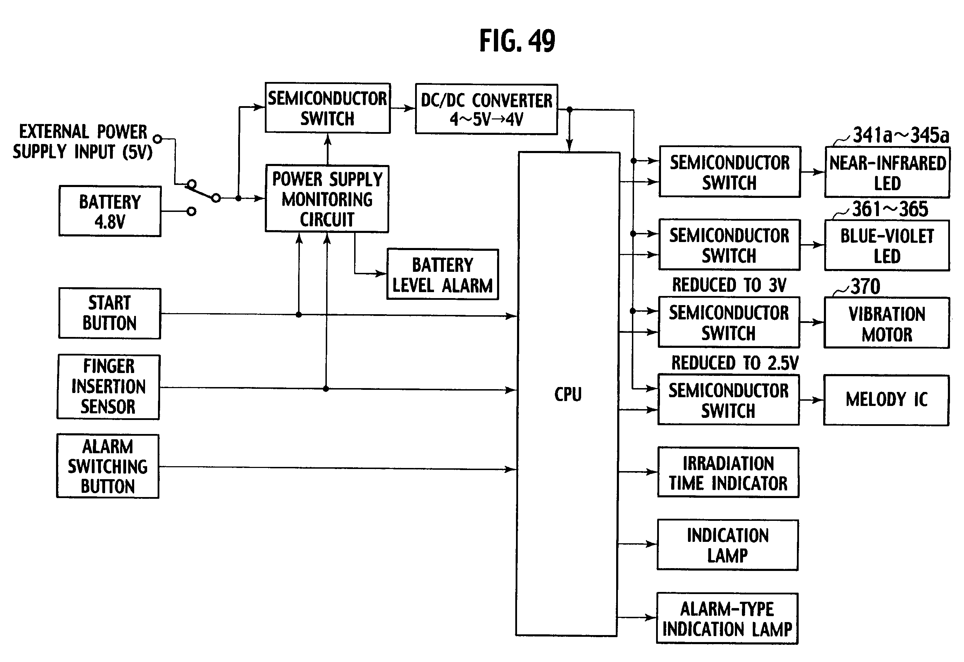

As shown in Fig. 32, a battery check LED and a mark 383 representing the battery check LED, a power ON display LED and a mark 384 representing the power ON display LED, and a melody/vibration switch display LED and a mark 385 representing the melody/vibration switch display LED as well as an AC adapter, not shown, are provided on a side of the upper case 310U. Fig. 49 is a block diagram of the fingertip stimulating apparatus 300.

When the user inserts his or her five fingers into the respective fingertip insertion units 331, 332, 333, 334, and 335, the photosensor 375 detects the insertion of the thumb and a power switch is turned on, whereby the fingertip stimulating apparatus 300 configured as described above starts the preset sequence operation in one cycle.

Namely, the near-infrared LEDs 341a, 342a, 343a, 344a, and 345a of the LED units 341, 342, 343, 344, and 345 emit pulses and irradiate near-infrared rays onto the borders of the fingernails of the respective fingertips of the user.

Furthermore, the blue-violet LEDs 361, 362, 363, 364, and 365 emit pulses, and blue-violet lights diffused upward from the openings 311, 312, 313, 314, and 315 of the upper case 310U stimulate the visual sensation of the user.

The blue-violet pulses are preferably light pulses at or near 3 Hz. As long as this condition is met, the light is not limited to the blue-violet light but may be, for example, a red light. Namely, blue-violet light generating members represented as the blue-violet LEDs 361, 362, 363, 364, and 365 can be defined as light pulse generating members.

Moreover, the vibration unit 370 vibrates the respective fingertips of the user. By vibrating (rubbing) the fingertips, it is expected to cause dilation of blood vessels.

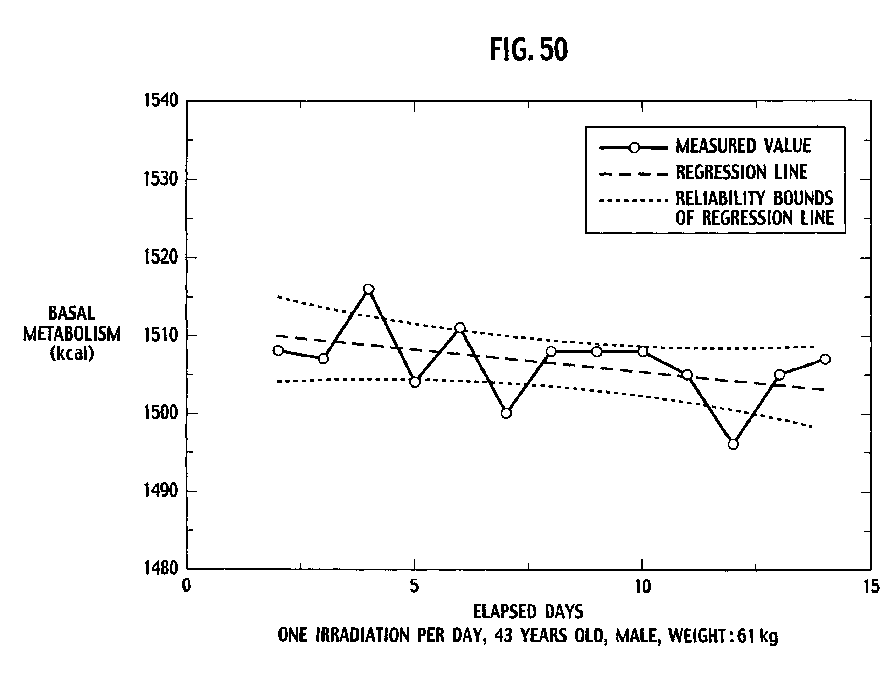

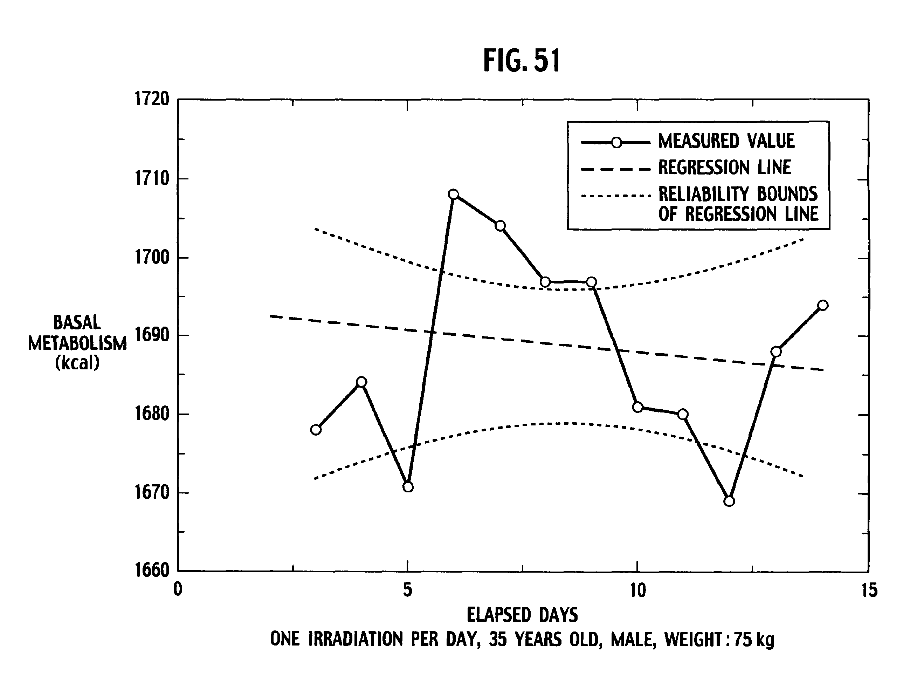

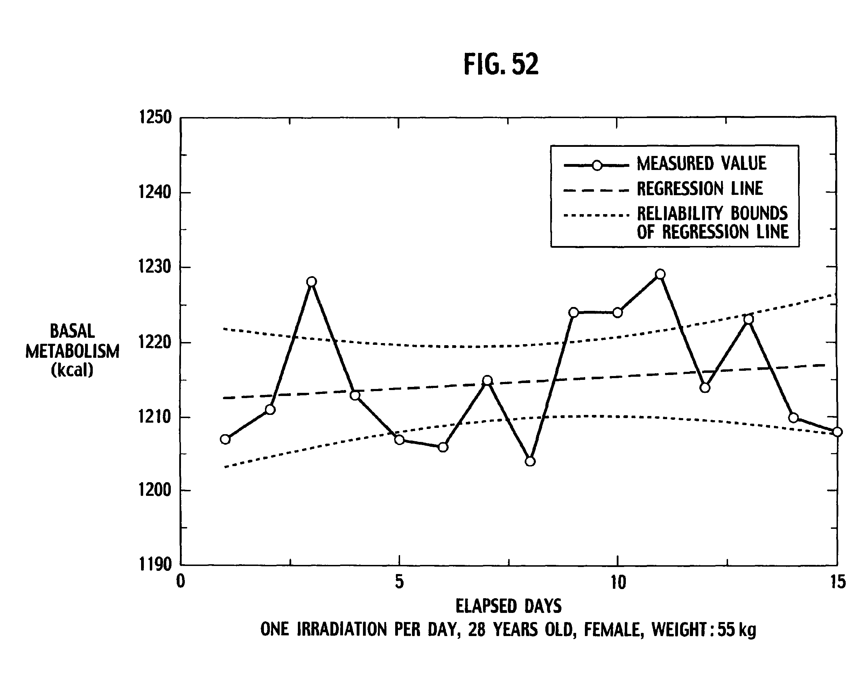

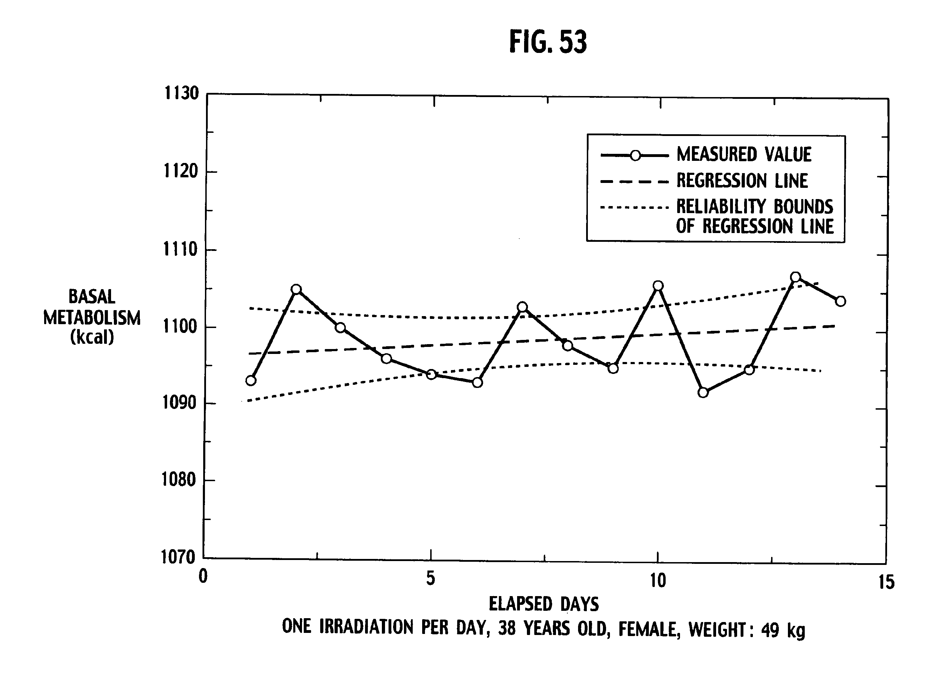

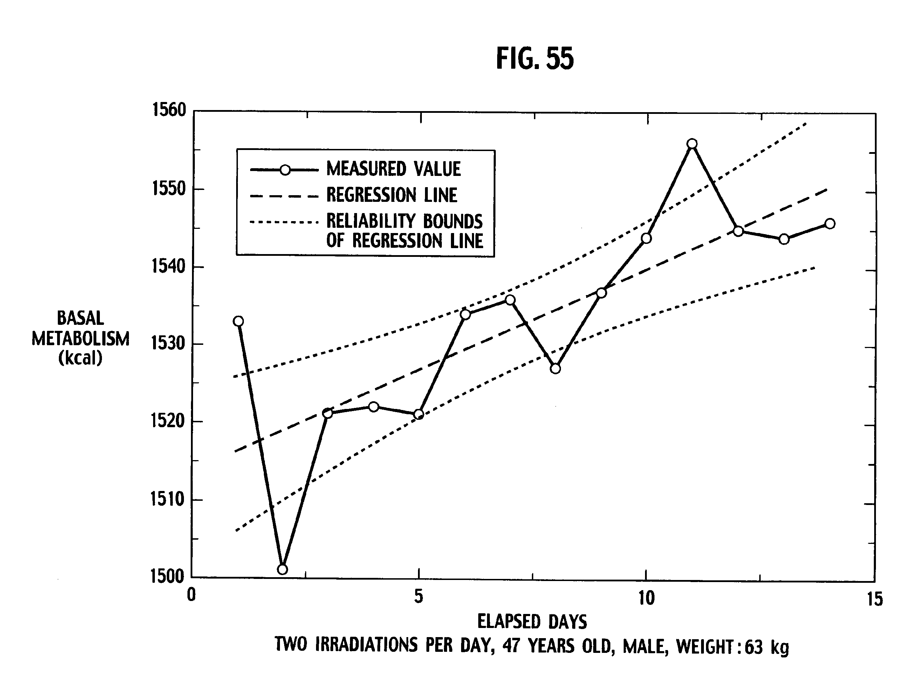

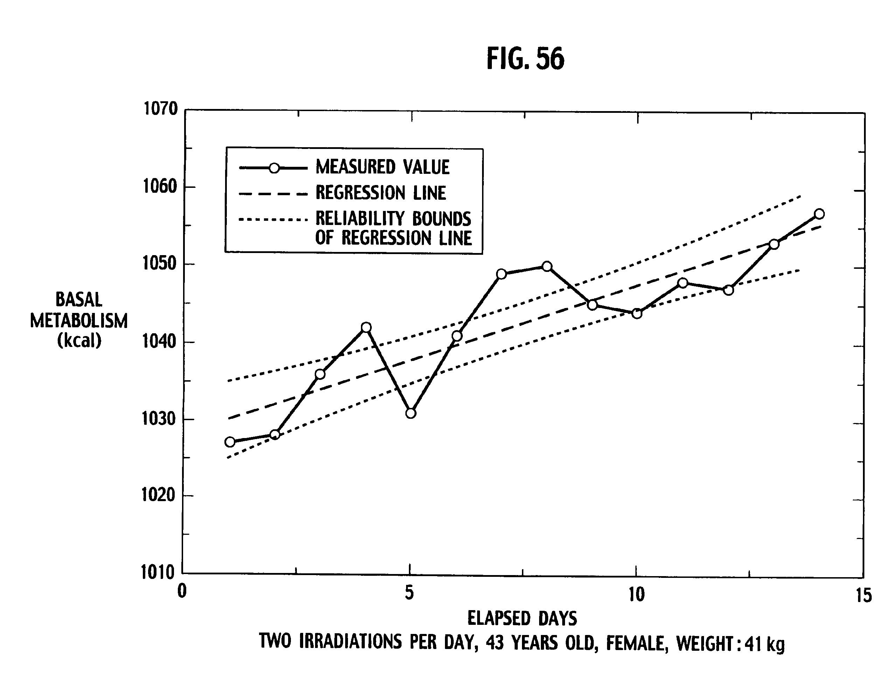

Figs. 50 to 56 are graphs showing experiments conducted to seven persons being tested about transition in basal metabolism for about two weeks using the fingertip stimulating apparatus 300 to validate the effects of the present invention, respectively.

The persons being tested are classified into a group of one irradiation per day and a group of two irradiations per day. Results of the two groups are shown in Figs. 50 to 53 and Figs. 54 to 56, respectively.

A "broken line" indicates a regression line, and a "dotted line" indicates a 95% reliability bound of the regression line.

The regression line is possibly arranged, relative to a population, in various manners within a range surrounded by the reliability bounds.

For each of the persons being tested in the group of one irradiation per day (Figs. 50 to 53), it is understood that a "gradient" of the regression line within the reliability bounds could be either "positive" or "negative", and it is judged that no significant increase is present.

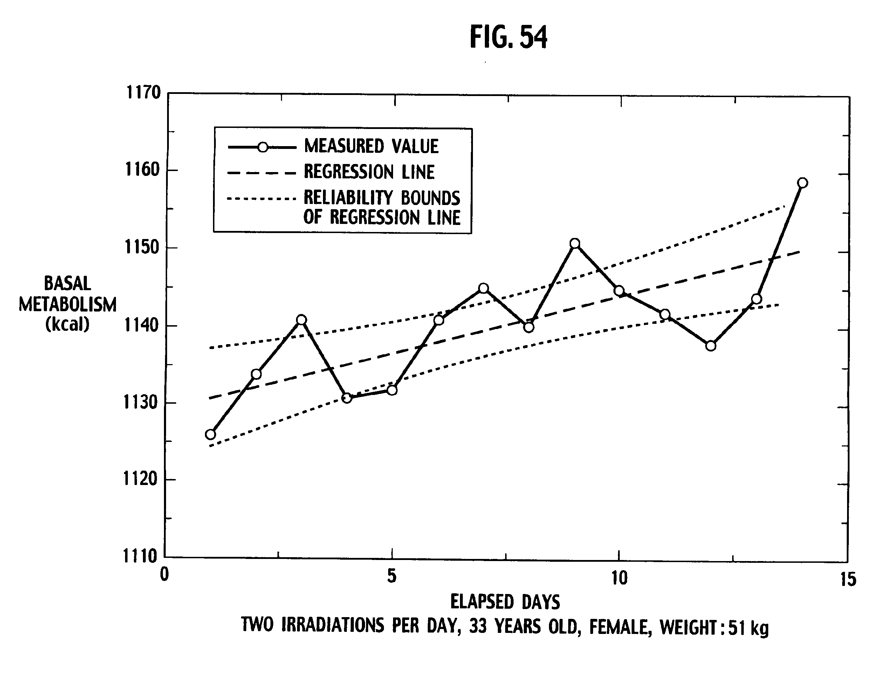

For each of the persons being tested in the group of two irradiations per day (Figs. 54 to 56), a basal metabolism tends to increase at sharp angle. A gradient of the regression line within the reliability bounds is always "positive" and it is judged that a significant increase is present.

It is understood from the above-stated results of experiments that if the fingertip stimulating apparatus 300 is used two or more times a day, a basal metabolism accelerating effect is produced.

One cause for the increased basal metabolism is considered as follows. The fingertip stimulation generated by irradiating the near-infrared rays onto regions near the borders of the fingernails of the fingertips using the fingertip stimulating apparatus 300 provides a safe and effective stimulus to the autonomic nerves to thereby control the body temperature.

Furthermore, another cause for the increased basal metabolism is considered as follows. By effectively providing visual stimulation as well as the fingertip stimulation using the fingertip stimulating apparatus 300, the body temperature is regulated and peripheral blood flow is thereby increased.

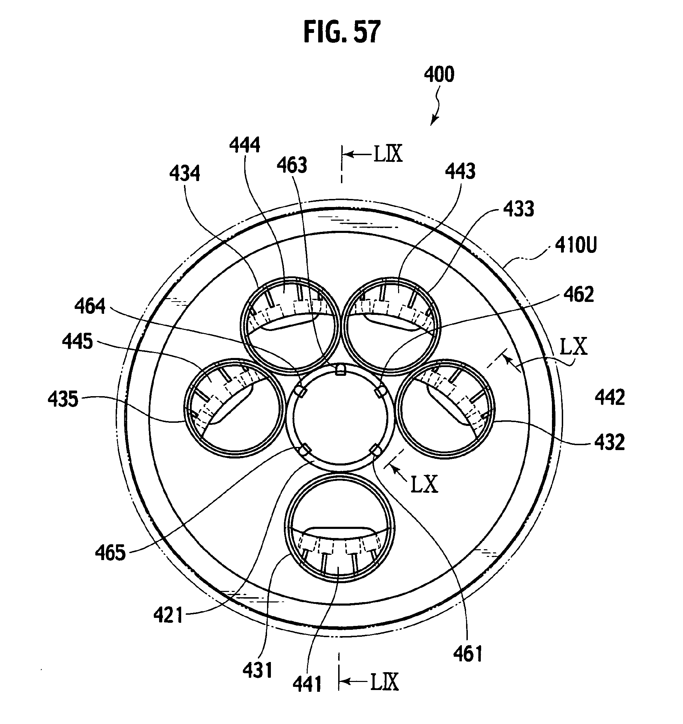

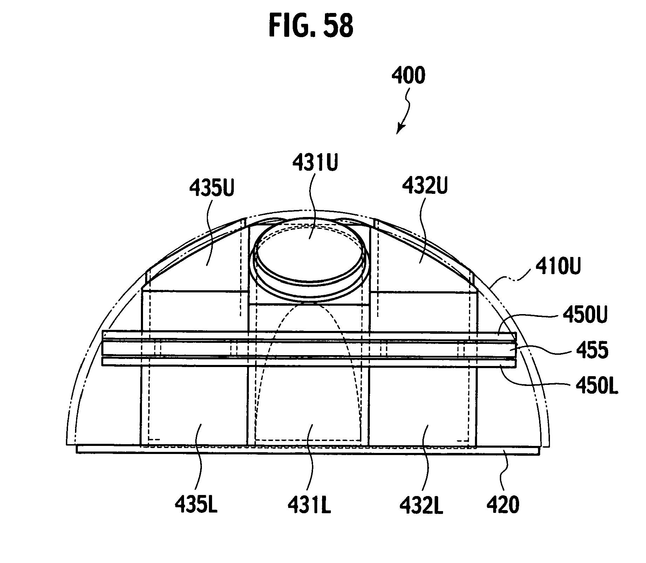

Figs. 57 to 60 show a sixth embodiment of a fingertip stimulating apparatus according to the present invention.

A fingertip stimulating apparatus 400 shown therein is constituted substantially spherically by an upper case 410U and a lower case 410L (not shown) that are semispherical and detachable from each other similarly to the fingertip stimulating apparatus 300 according to the fifth embodiment.

The fingertip stimulating apparatus 400 differs from the fingertip stimulating apparatus 300 in that fingertip insertion units 431, 432, 433, 434, and 435 are divided into upper fingertip insertion units 431U, 432U, 433U, 434U, and 435U and lower fingertip insertion units 431L, 432L, 433L, 434L, and 435L, respectively.

The upper fingertip insertion units 431U, 432U, 433U, 434U, and 435U are integrally fixed to an opening of the upper case 410U.

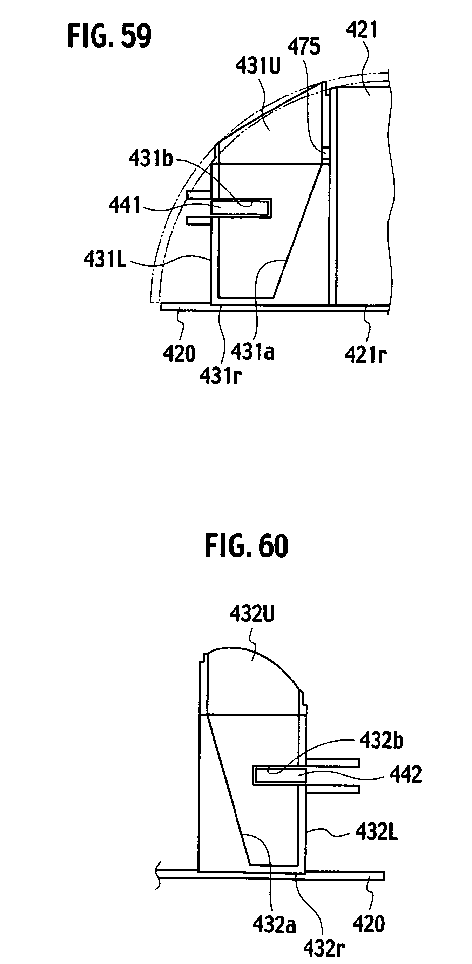

On the other hand, the lower fingertip insertion units 431L, 432L, 433L, 434L, and 435L are substantially cylindrical units. As shown in Fig. 59, the lower fingertip insertion unit 431L is held to be rotatable about an axis line while a circular bottom of the lower fingertip insertion unit 431L is positioned into a circular concave portion 431r formed in a support plate 420.

As shown in Fig. 60, the lower fingertip insertion unit 432L is held to be rotatable about an axis line while a circular bottom of the lower fingertip insertion unit 432L is positioned into a circular concave portion 432r formed in the support plate 420. Likewise, although not shown in the drawings, the remaining lower fingertip insertion units 433L, 434L, and 435L are held to be rotatable about axis lines while circular bottoms of the lower fingertip insertion units 433L, 434L, and 435L are positioned into circular concave portions 433r, 434r, and 435r (not shown) formed in the support plate 420, respectively.

As shown in Fig. 59, an inclined guide unit 431a for guiding an inserted fingertip in a certain direction is formed in the lower fingertip insertion unit 431L. A window hole 431b is formed on an opposite side to the guide unit 431a. An LED unit 441 is arranged in the window hole 431b.

As shown in Fig. 60, an inclined guide unit 432a for guiding an inserted fingertip in a certain direction is formed in the lower fingertip insertion unit 432L. A window hole 432b is formed on an opposite side to the guide unit 432a. An LED unit 442 is arranged in the window hole 432b.

Likewise, although not shown in the drawings, inclined guide units 433a, 434a, 435a for guiding inserted fingertips in certain directions are formed in the lower fingertip insertion units 433L, 434L, and 435L, respectively. Window holes 433b, 434b, and 435b (not shown) are formed on opposite sides to the guide units 433a, 434a, and 435a, respectively. LED units 443, 444, and 445 are arranged in the respective window holes 433b, 434b, and 435b.

Such lower fingertip insertion units 431L, 432L, 433L, 434L, and 435L including the LED units 441, 442, 443, 444, and 445 are rotatable about the respective axis lines. Due to this, when the fingertips are inserted, arbitrary lower fingertip insertion units among the lower fingertip insertion units 431L, 432L, 433L, 434L, and 435L can be rotated by necessary angles so as to match to directions of the inserted fingertips, respectively.

In this manner, directions of the LED units 441, 442, 443, 444, and 445 can be easily adjusted to those of the corresponding fingertips. Therefore, if near-infrared LEDs 441a, 442a, 443a, 444a, and 445a (not shown) are activated, near-infrared rays are efficiently irradiated onto borders of fingernails of the respective fingertips of a user in appropriate directions.

The fingertip stimulating apparatus 400 configured as described above executes a preset sequence operation in one cycle similarly to the fingertip stimulating apparatus 300 according to a fifty-first embodiment.

Similarly to the fingertip stimulating apparatus 300, it is understood that the fingertip stimulating apparatus 400 can provide a safe and effective stimulus to the autonomic nerves by fingertip stimulation and control the body temperature.

Moreover, it is understood that the body temperature is regulated to thereby accelerate increase of peripheral blood flow by effectively providing visual stimulation as well as the fingertip stimulation using the fingertip stimulating apparatus 400.

Furthermore, it is understood that a basal metabolism accelerating effect is produced by using the fingertip stimulating apparatus 400.

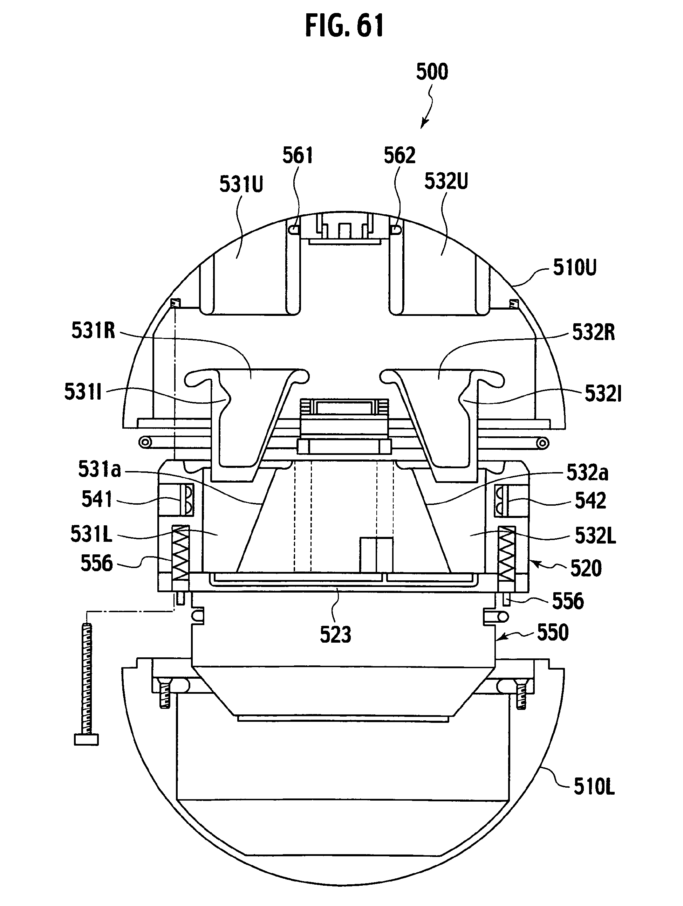

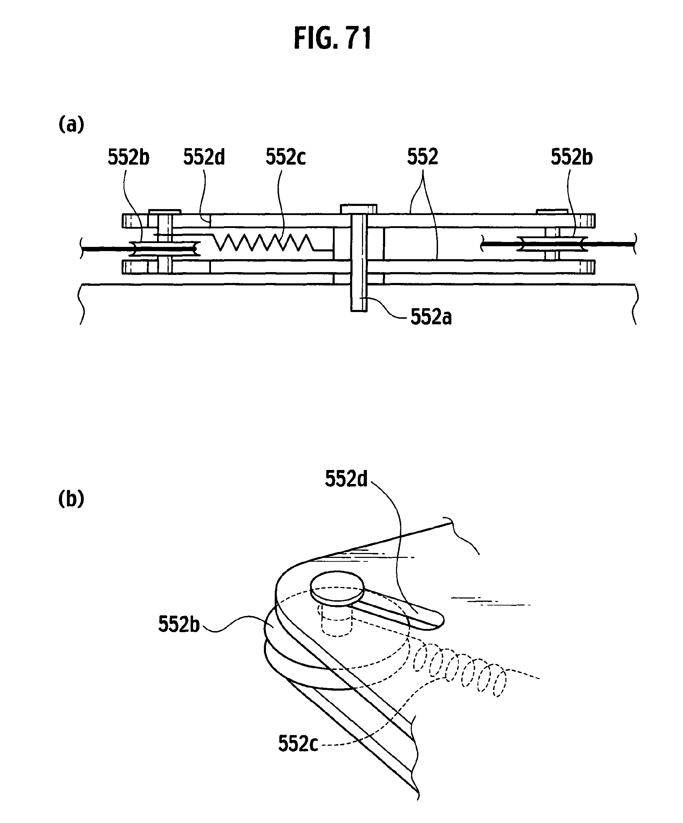

Figs. 61 to 76 show a seventh embodiment of a fingertip stimulating apparatus according to the present invention.

As shown in Fig. 61, a fingertip stimulating apparatus 500 is constituted substantially spherically by an upper case 510U and a lower case 510L that are detachable from each other and semispherical. A main housing 520 is arranged in the fingertip stimulating apparatus 500.

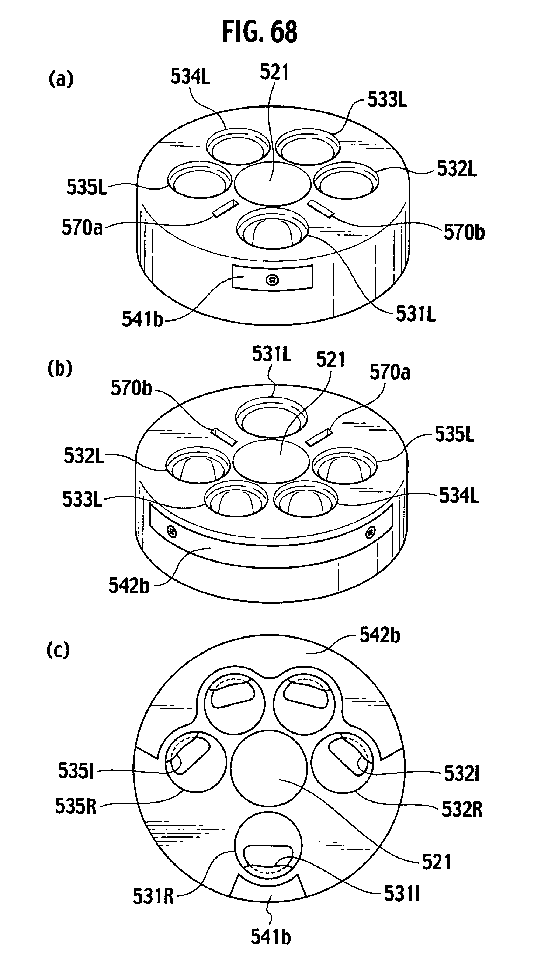

As shown in Fig. 61, fingertip insertion units into which five fingertips are inserted are divided into upper fingertip insertion units 531U to 535U and lower fingertip insertion units 531L to 535L, respectively. These upper and lower fingertip insertion units are arranged as shown in Fig. 68. However, for the sake of convenience, Fig. 61 shows that the upper fingertip insertion units 531U and 532U are opposed to each other, and the lower fingertip insertion units 531L and 532L are opposed to each other.

The upper fingertip insertion units 531U to 535U (533U, 534U, and 535U are not shown in the drawing) are integrally fixed to corresponding openings of the upper case 510U. The lower fingertip insertion units 531L to 535L are provided in the main housing 520.

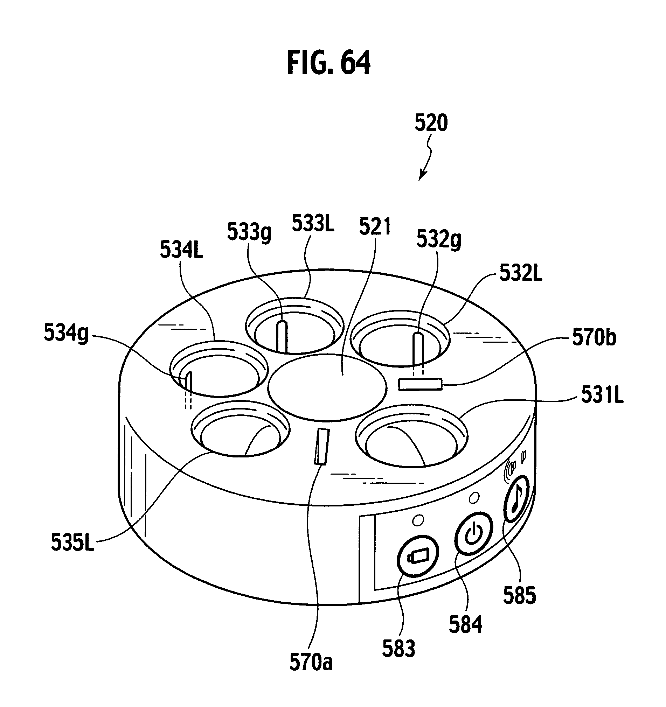

As shown in Fig. 64, a pump storage unit 521 is formed at the center of the main housing 520. The five lower fingertip insertion units 531L to 535L are formed around the pump storage unit 521.

One lower fingertip insertion unit 531L as well as the corresponding upper fingertip insertion unit 531U (which shall apply and, therefore omitted hereinafter) is a unit into which a fingertip of a thumb is inserted, and is formed into a cylindrical shape larger in diameter than the other four lower fingertip insertion units 532L, 533L, 534L, and 535L. The other four lower fingertip insertion units 532L, 533L, 534L, and 535L are units into which four fingertips except for the thumb are inserted, respectively. The other four lower fingertip insertion units 532L, 533L, 534L, and 535L are formed into cylindrical shapes equal in diameter so as to be shared between the right hand and the left hand.

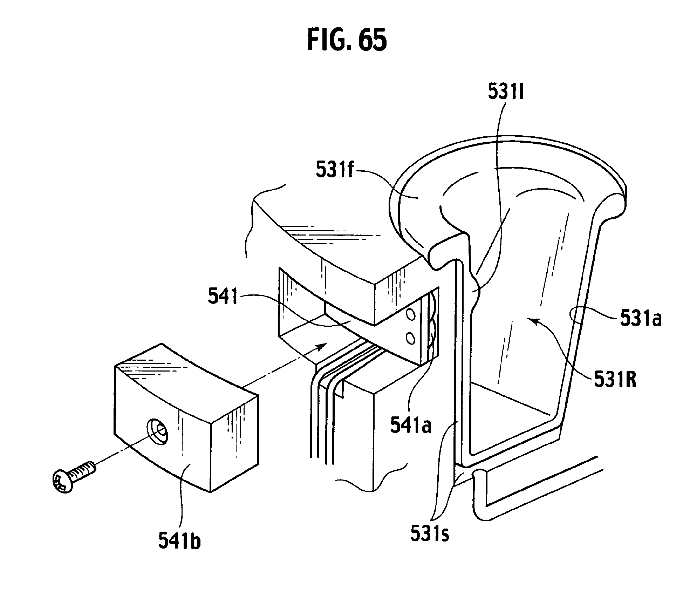

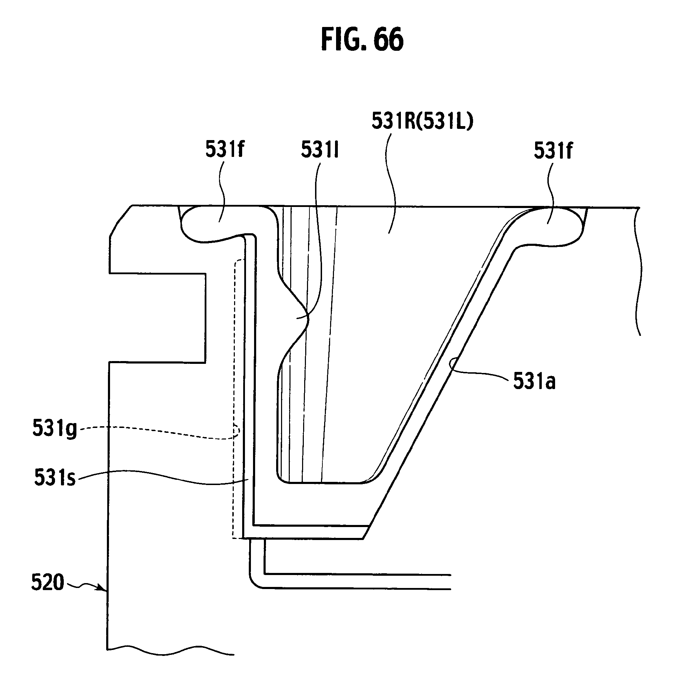

As shown in Figs. 65 and 66, an inclined guide unit 531a for guiding the inserted fingertip in a certain direction is formed in a lower portion of the lower fingertip insertion unit 531L. Although not shown in the drawings, inclined guide units 532a, 533a, 534a, and 535a (not shown) for guiding the inserted fingertips in certain directions are formed in lower portions of the other lower fingertip insertion units 532L, 533L, 534L, and 535L, respectively.

As shown in Fig. 68, the guide unit 531a for the lower fingertip insertion unit 531L for the thumb guides the fingertip in an extension direction of a line extending from a center of the pump storage unit 521 to a center of the lower fingertip insertion unit 531L for the thumb.

Further, the guide units 532a, 533a, 534a, and 535a for the lower fingertip insertion units 532L, 533L, 534L, and 535L for the fingers other than the thumb guide the fingertips in extension directions of lines extending from the center of the lower fingertip insertion unit 531L for the thumb to centers of the lower fingertip insertion units 532L, 533L, 534L, and 535L, respectively.

Since directions of these guide units 531a, 532a, 533a, 534a, and 535a are similar to contents shown in Fig. 44 in relation to the fingertip stimulating apparatus 300 according to the fifth embodiment, reference is made to the contents shown in Fig. 44.

As shown in Fig. 65, an LED unit 541 is arranged at a predetermined height on an opposite side to the guide unit 531a, and fixed to the position by a fastener 541b. Although not shown in the drawings, LED units 542, 543, 544, and 545 (not shown) are arranged at predetermined heights on opposite sides to the guide units 532a, 533a, 534a, and 535a, respectively, and fixed to the positions by a common fastener 542b (see Fig. 68). These LED units 541, 542, 543, 544, and 545 are arranged at the same height. The height is designed to a height at which borders of fingernails of fingers are located when the fingers are inserted into the respective lower fingertip insertion units 531L, 532L, 533L, 534L, and 535L.

As shown in Figs. 65 and 66, a rubber fingerstall 531R made of an elastic member transmitting a near-infrared ray and flexible is stored in the lower fingertip insertion unit 531L. Likewise, although not shown in the drawings, silicon rubber fingerstalls 532R, 533R, 534R, and 535R (not shown) are stored in the lower fingertip insertion units 532L, 533L, 534L, and 535L, respectively. It is preferable to use silicon rubber as the elastic member, that is, it is preferable to use silicon rubber fingerstalls.

The rubber fingerstall 531R is of a shape closely attached to the guide unit 531a along inclination of the guide unit 531a. Shapes and dimensions of a side surface on an LED unit 541 side (peripheral surface) and a bottom of the rubber fingerstall 531R are designed so as to form an airtight space 531s between a corresponding side surface (peripheral surface) and a corresponding bottom of the lower fingertip insertion unit 531L. Although not shown in the drawings, shapes and dimensions of the other rubber fingerstalls 532R, 533R, 534R, and 535R are designed similarly. Due to this, the other rubber fingerstalls 532R, 533R, 534R, and 535R are closely attached to the guide units 532a, 533a, 534a, and 535a along inclinations of the guide units 532a, 533a, 534a, and 535a, respectively. Further, shapes and dimensions of side surfaces on LED units 542, 543, 544, and 545 sides (peripheral surfaces) and bottoms of the rubber fingerstalls 532R, 533R, 534R, and 535R are designed so as to form airtight spaces 532s, 533s, 534s, and 535s (not shown) between corresponding side surfaces (peripheral surfaces) and corresponding bottoms of the lower fingertip insertion units 532L, 533L, 534L, and 535L, respectively.

As shown in Figs. 64 and 66, grooves 531g, 532g, 533g, 534g, and 535g preventing the rubber fingerstalls 531R, 532R, 533R, 534R, and 535R from being closely attached to wall surfaces of the lower fingertip insertion units 531L, 532L, 533L, 534L, and 535L are formed vertically long on side surfaces of the lower fingertip insertion units 531L, 532L, 533L, 534L, and 535L on which surfaces the airtight spaces 531s, 532s, 533s, 534s, and 535s are formed between the lower fingertip insertion units 531L, 532L, 533L, 534L, and 535L and the rubber fingerstalls 531R, 532R, 533R, 534R, and 535R, respectively.

The rubber fingerstall 531R includes a lens unit 5311 at a position corresponding to the LED unit 541. The lens unit 5311 functions to converge a near-infrared ray emitted from a near-infrared LED 541a of the LED unit 541 and to irradiate the near-infrared ray onto a border of a fingernail of the fingertip. The other rubber fingerstalls 532R, 533R, 534R, and 535R include lens units 5321, 5331, 5341, and 5351 that function similarly to the lens unit 5311 at positions corresponding to the LED units 542, 543, 544, and 545, respectively.

In the main housing 520, portions corresponding to at least the lens units 5311, 5321, 5331, 5341, and 5351 of the rubber fingerstalls 531R, 532R, 533R, 534R, and 535R, that is, portions corresponding to near-infrared LEDs 541a, 542a, 543a, 544a, and 545a (LED units 541, 542, 543, 544, and 545) are made of a material transmitting a near-infrared ray.

A wavelength of a near-infrared ray emitted from each of the near-infrared LEDs 541a, 542a, 543a, 544a, and 545a is preferably in a range from 820 nm to 920 nm, more preferably near 870 nm. It is also preferable to intermittently turn on or off the near-infrared LEDs 541a, 542a, 543a, 544a, and 545a so as to irradiate near-infrared rays onto regions near borders of the fingernails of the fingertips and to stimulate the fingertips, respectively.

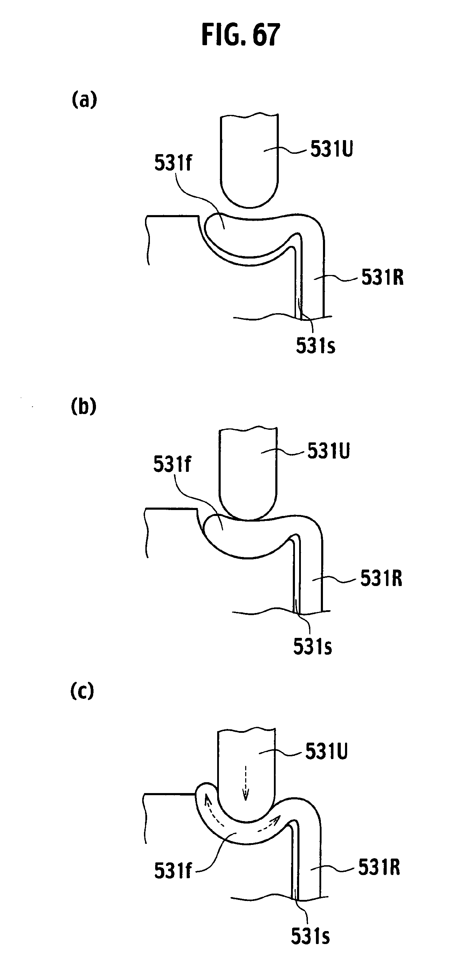

As shown in Fig. 67, to fix the rubber fingerstall 531R, a lower surface of the upper fingertip insertion unit 531U fixed to the upper case 510U presses a flange 531f of the fingerstall from above, thereby fixing the rubber fingerstall 531R to the lower fingertip insertion unit 531L. Likewise, although not shown in the drawings, lower surfaces of the upper fingertip insertion units 532U, 533U, 534U, and 535U fixed to the upper case 510U press flanges 532f, 533f, 534f, and 535f (not shown) of the fingerstalls from above, thereby fixing the rubber fingerstalls 532R, 533R, 534R, and 535R to the lower fingertip insertion units 532L, 533L, 534L, and 535L, respectively.

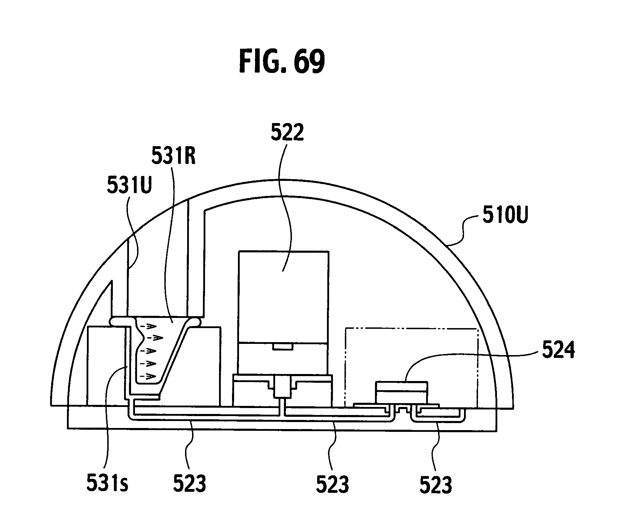

As shown in Fig. 69, the airtight space 531s is connected to a pump 522 disposed in the pump storage unit 521 via an air passage 523 formed in a lower portion of the main housing 520. Likewise, although not shown in the drawings, the other airtight spaces 532s, 533s, 534s, and 535s are connected to the pump 522 via air passages 523 formed in the lower portion of the main housing 520, respectively.

Namely, the pump 522 is connected to all the airtight spaces 531s, 532s, 533s, 534s, and 535s via the air passages 523. In addition, all the airtight spaces 531s, 532s, 533s, 534s, and 535s are connected to a solenoid valve 524 including a vent hole via the air passages 523.

Due to this, when a motor is driven to actuate the pump 522, pressurized air is fed into the airtight spaces 531s, 532s, 533s, 534s, and 535s to enlarge the airtight spaces 531s, 532s, 533s, 534s, and 535s, thereby deforming the rubber fingerstalls 531R, 532R, 533R, 534R, and 535R to envelope the respective fingers. It is thereby possible to closely attach the lens units 5311, 5321, 5331, 5341, and 5351 to positions of the borders of the fingernails of the respective fingers. At this time, a motor current for the pump 522 can be monitored, pressurization can be stopped at an optimum pressure, and the pressurization state can be maintained.

In this pressurization state, near-infrared rays emitted from the near-infrared LEDs 541a, 542a, 543a, 544a, and 545a of the LED units 541, 542, 543, 544, and 545 are appropriately irradiated onto the borders of the fingernails of the fingers through the lens units 5311, 5321, 5331, 534l, and 5351 of the rubber fingerstalls 531R, 532R, 533R, 534R, and 535R, respectively.

If the pressurization of the rubber fingerstalls 531R, 532R, 533R, 534R, and 535R is to be stopped, the solenoid valve 524 is activated. By doing so, the pressurized air within the airtight spaces 531s, 532s, 533s, 534s, and 535s is evacuated.

Moreover, while pressurization and pressurization stop by the pump 522 and evacuation by the solenoid valve 524 are set as one cycle, cycles are repeated. It is thereby possible to press and rub the fingertips.

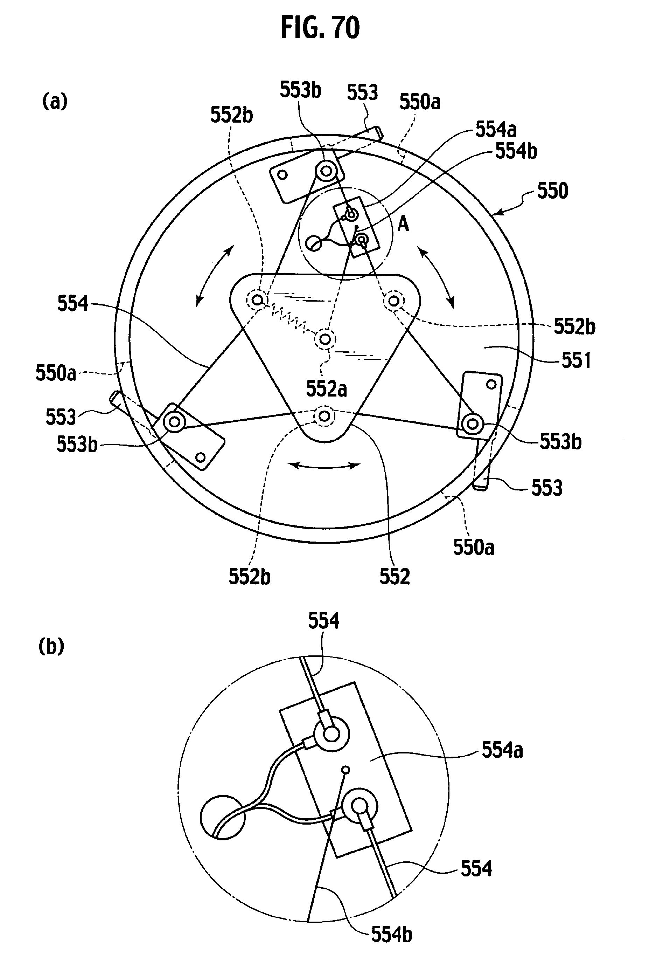

As shown in Figs. 70 to 72, an attachment/detachment mechanism arranged in a lower housing 550 integrally fixed to a lower portion of the main housing 520 realizes attachment or detachment of the upper case 510U to or from the lower case 510L.

The attachment/detachment mechanism includes a base plate 551, and a rotation plate 552 attached to a center of the base plate 551 so that the rotation plate 552 is rotatable about a rotation shaft 552a.

Lock pins 553 are attached to three portions around the base plate 551 so as to be able to oscillate. The lock pins 553 are designed to protrude from an opening 550a formed in a peripheral surface of the lower housing 550 to a predetermined position by a biasing force of a coil spring 553a.

A Biometal 554 is wound around pulleys 553b of each lock pin 553 and pulleys 552b of the rotation plate 552. Both ends of the Biometal 554 are fixed to a terminal plate 554a. Because the terminal plate 554a is not fixed to the base plate 551, the terminal plate 554a is connected to the rotation shaft 552a of the rotation plate 552 by a wire cable 554b.

One of the pulleys 552b of the rotation plate 552 is biased in a direction of attracting the Biometal 554 along an elongate hole 552d by a spring 552c, thereby preventing the Biometal 554 from being slackened.

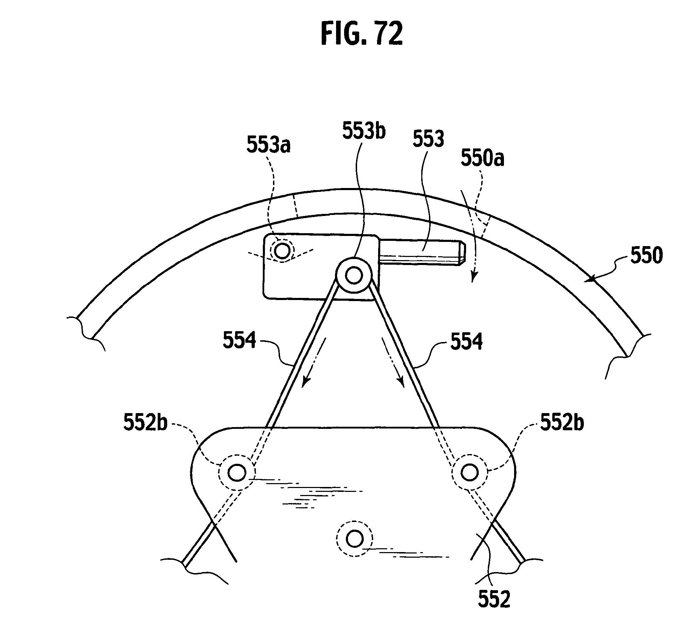

If current is carried across the Biometal 554, the Biometal 554 is contracted and shortened, whereby the lock pins 553 are oscillated and stored from the opening 550a formed in the peripheral surface of the lower housing 550 in the lower housing 550 as shown in Fig. 72.

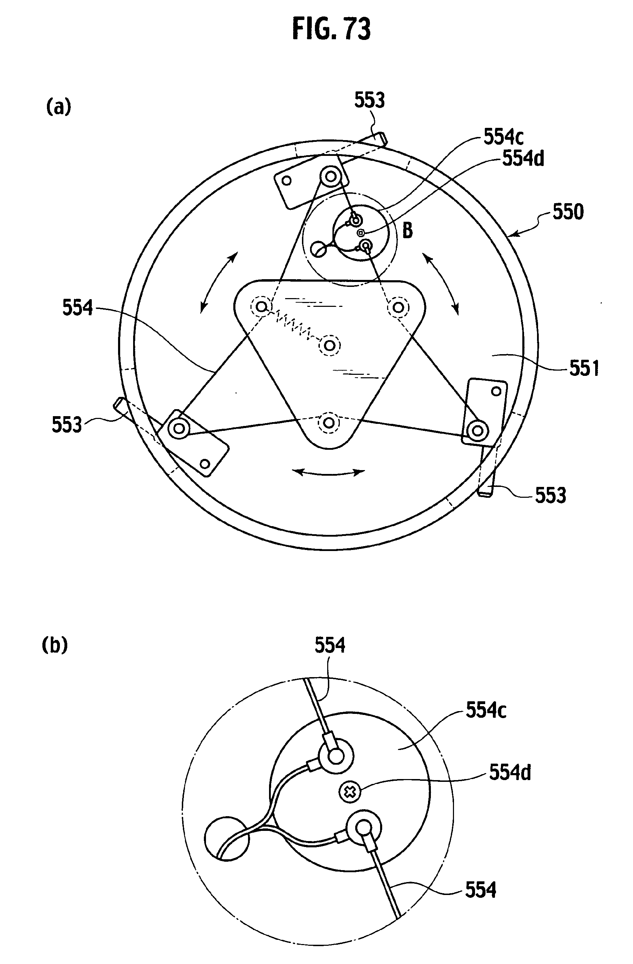

Alternatively, the terminal plate 554a of the Biometal 554 can be replaced by a terminal plate 554c shown in Fig. 73 and configured to be fixed to the base plate 551 by a screw 554d. In this case, it is possible to dispense with the wire cable.

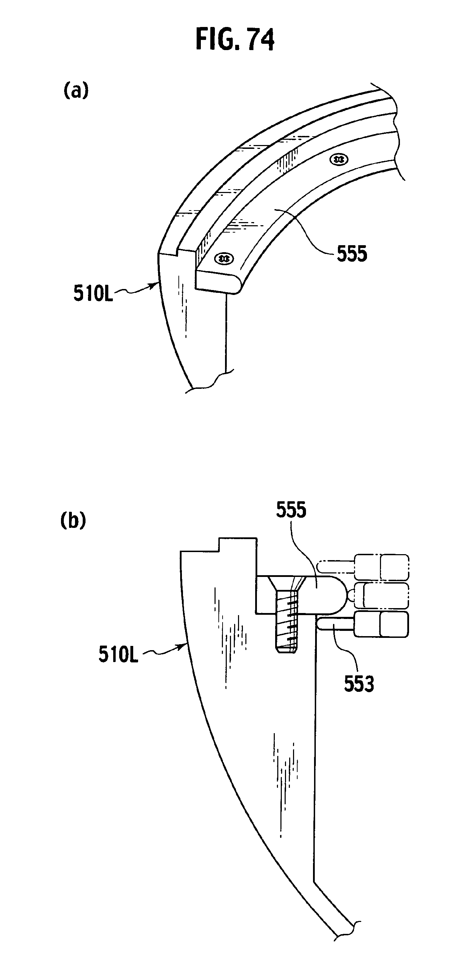

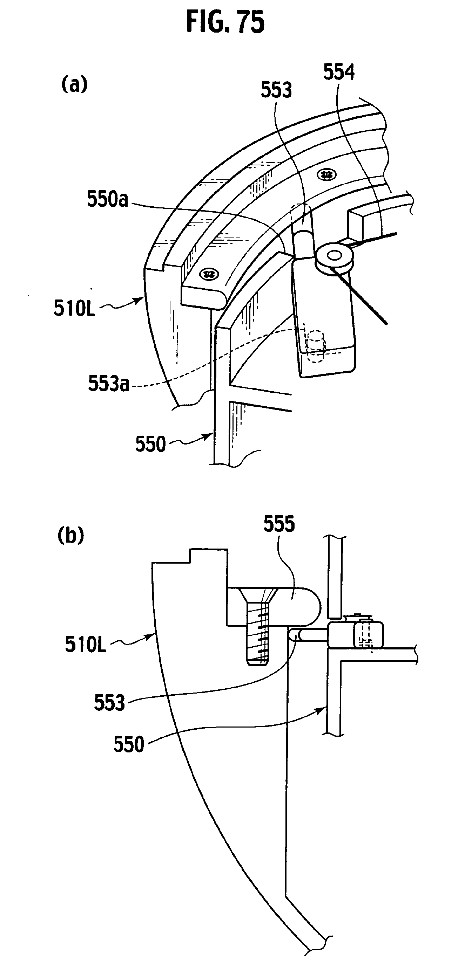

As shown in Figs. 74 to 76, the upper case 510U is attached to or detached from the lower case 510L. Namely, when the upper case 510U is attached to the lower case 510L, each of the lock pins 553 is located below a lock ring 555 of the lower case 510L, protruded outward from the opening 550a formed in the peripheral surface of the lower housing 550 by the biasing force of a coil spring 553a, and forced into a position below the lock ring 555 as shown in Fig. 75.

On the other hand, if the upper case 510U is to be detached from the lower case 510L, then current is carried across the Biometal 554, and the lock pins 553 are oscillated and stored in the lower housing 550 from the opening 550a formed in the peripheral surface of the lower housing 550.

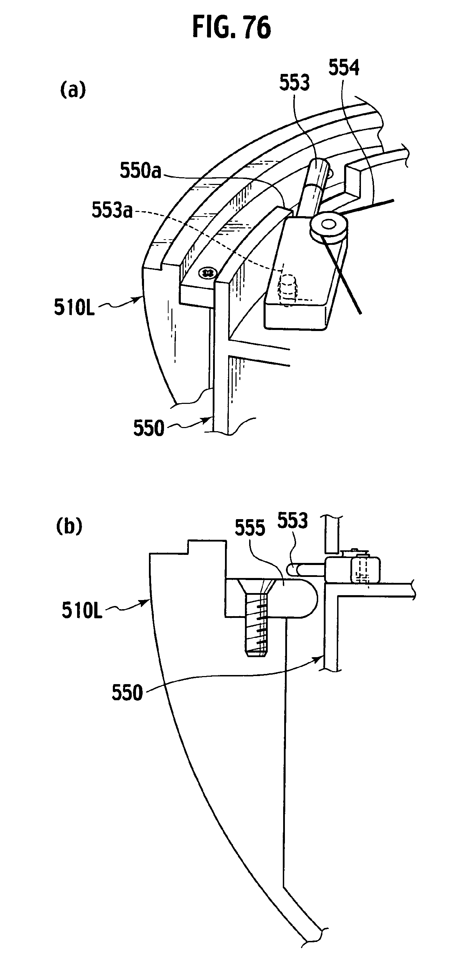

At this time, as shown in Fig. 61, a depression plunger 556 provided in the main housing 520 depresses the lower case 510L. As a result, the lock ring 555 of the lower case 510L is moved downward to release a lock state as shown in Figs. 74 and 76.

Moreover, as shown in Fig. 61, the fingertip stimulating apparatus 500 includes five blue-violet LEDs 561 to 565 (only the blue-violet LEDs 561 and 562 are shown) near upper ends of the upper fingertip insertion units 531U to 535U.

A wavelength of a blue-violet light emitted from each of the blue-violet LEDs 561 to 565 is preferably in a range of 390 nm to 420 nm, more preferably near 405 nm.

It is also preferable to intermittently turn on or off the blue-violet LEDs 561 to 565 so as to stimulate visual sensation of the user.

When the blue-violet LEDs 561 to 565 are activated, blue-violet lights are diffused upward from the respective openings of the upper case 510U to which the upper fingertip insertion units 531U to 535U are fixed and visually recognized by the user.

As shown in Figs. 64 and 68, the fingertip stimulating apparatus 500 includes a vibration unit (vibrators 570a and 570b) in the main housing 520. The vibrators 570a and 570b are substantially disk-shaped and produce vibrations by being rotated about their central axes, and arranged with the central axes orthogonal to each other. Due to this, when the vibrators 570a and 570b operate, a whizzing sound is produced.

Although not shown in the drawings, a photosensor that detects insertion of a fingertip when the fingertip is inserted is provided in each of arbitrary fingertip insertion units among the upper fingertip insertion units 531U to 535U, e.g., the upper fingertip insertion unit 531U for the thumb and any one of the remaining upper fingertip insertion units. When the user inserts his or her fingertips into the respective upper fingertip insertion units 531U to 535U, the photosensors detect insertion of the fingertips. The detection is used as a start condition for a preset operation sequence in one cycle of the fingertip stimulating apparatus 500.

As shown in Fig. 64, a battery check LED and a mark 583 representing the battery check LED, a power ON display LED and a mark 584 representing the power ON display LED, and a melody/vibration switch display LED and a mark 585 representing the melody/vibration switch display LED are provided on a side of the main housing 520. The LEDs and the marks 583, 584, and 585 are visually recognizable from outside through the light-transmission side of the upper case 510U.

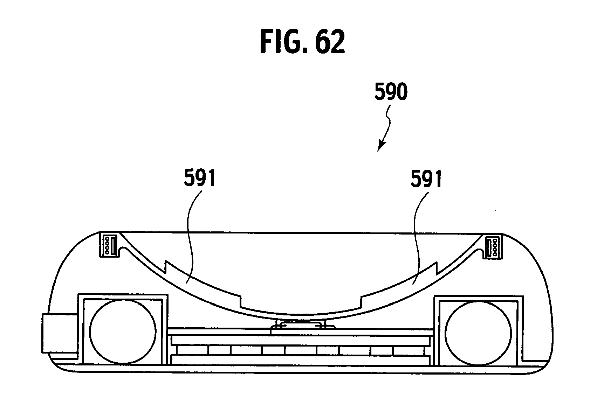

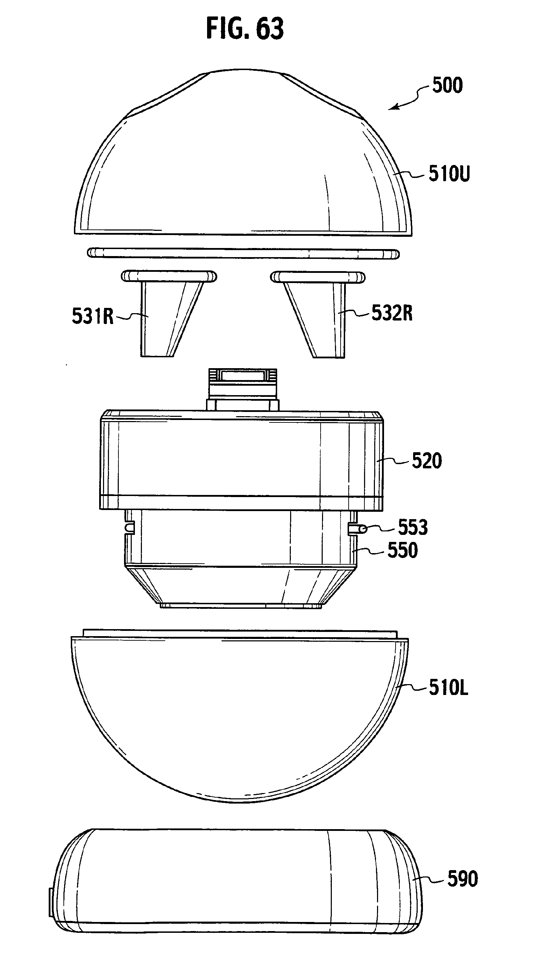

As shown in Figs. 62 and 63, the fingertip stimulating apparatus 500 includes a cradle 590 on which the fingertip stimulating apparatus 500 is mounted. The cradle 590 includes convex portions 591 fitted into the upper fingertip insertion units 531U to 535U of the upper case 510U when the fingertip stimulating apparatus 500 is mounted on the cradle 590 while inverted upside down.

Namely, if the fingertip stimulating apparatus 500 is mounted on the cradle 590 while inverted upside down, then electric power for the Biometal 554 is supplied from a cradle 590 side to a fingertip stimulating apparatus 500 side, the Biometal 554 is contracted, and the lock state is released by the depression plunger 556. As a result, a battery disposed in the lower housing 550 can be replaced by another battery.

Similarly, it is understood that the fingertip stimulating apparatus 500 can provide a safe and effective stimulus to the autonomic nerves by fingertip stimulation and control the body temperature.

Moreover, it is understood that the body temperature is regulated to thereby accelerate increase of peripheral blood flow by effectively providing visual stimulation as well as the fingertip stimulation using the fingertip stimulating apparatus 500.

Furthermore, it is understood that a basal metabolism accelerating effect is produced by using the fingertip stimulating apparatus 500.

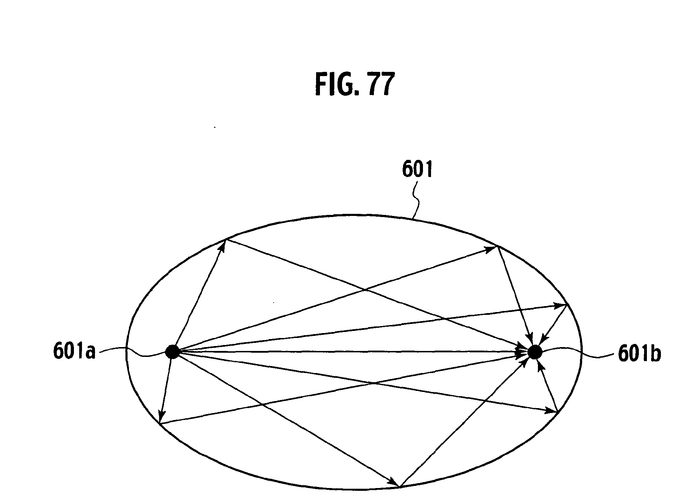

Figs. 77 to 79 show an eighth embodiment of a fingertip stimulating apparatus according to the present invention.

A fingertip stimulating apparatus 600 shown therein employs elliptically cylindrical mirrors 601. As shown in Fig. 77, a near-infrared ray is irradiated onto a position of a border of a fingernail of a fingertip using a property of the elliptically cylindrical mirror 601 that lights emitted from one focal point 601a are all focused on the other focal point 601b.

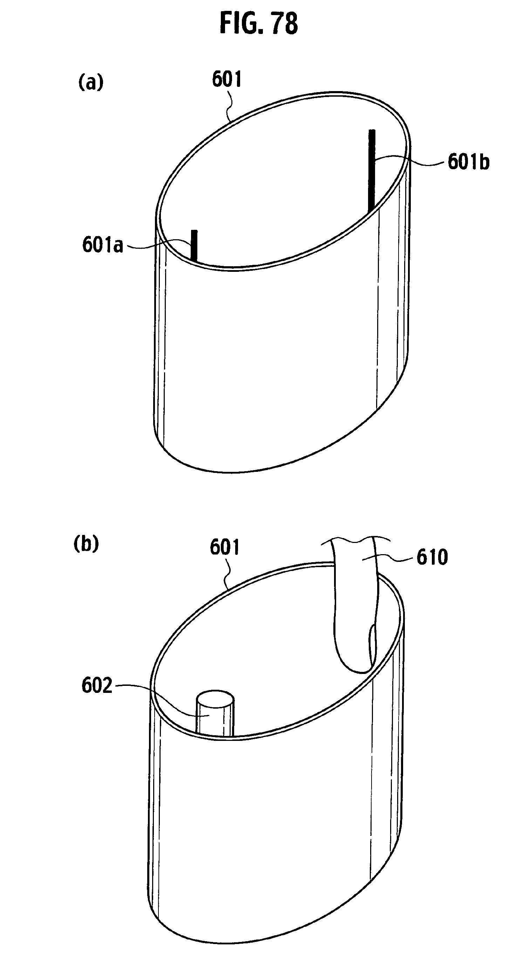

Namely, as shown in Fig. 78, a near-infrared LED 602 is arranged (see Fig. 78(b)) at a position of one focal point 601a (see Fig. 78 (a)), and a fingertip 610 is arranged (see Fig. 78 (b)) at a position of the other focal point 601b (see Fig. 78 (a)).

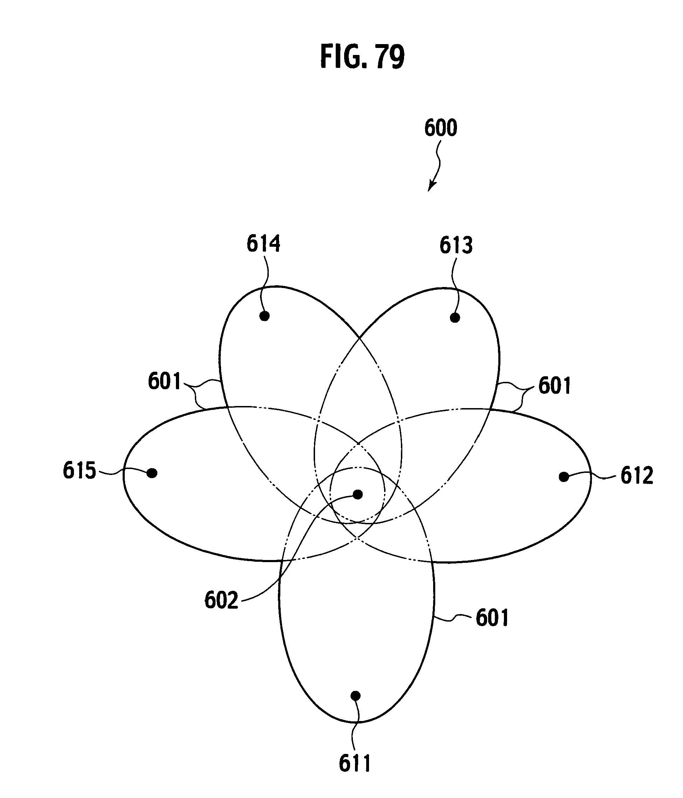

As shown in Fig. 79, the fingertip stimulating apparatus 600 is configured so that the near-infrared LED 602 is arranged while one focal point of each of five elliptically cylindrical mirrors 601 are made common to the five elliptically cylindrical mirrors 601, and a thumb and the other four fingers are put onto other focal points 611, 612, 613, 614, and 615, respectively. The fingertip stimulating apparatus 600 can thereby irradiate near-infrared rays onto positions of the borders of the fingernails of fingertips of all five fingers of one hand, respectively.

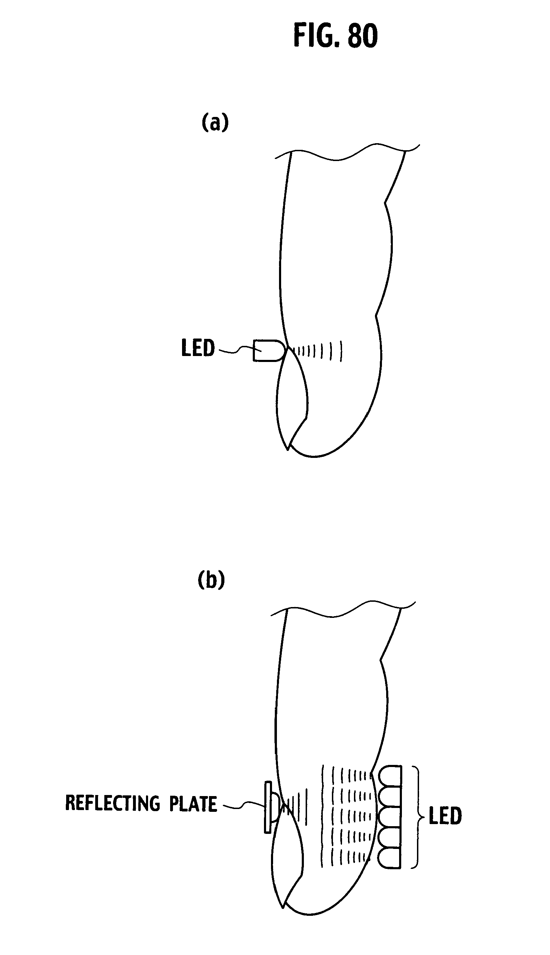

As shown in Fig. 80 (a), each of the above-stated fingertip stimulating apparatuses 300, 400, 500, and 600 irradiates a near-infrared ray onto the position of the border of the fingernail of the fingertip by arranging each near-infrared LED on a fingernail side of the fingertip.

Alternatively, as shown in Fig. 80(b), a near-infrared ray can be irradiated onto the position of the border of the fingernail of the fingertip by a reflection method by arranging the near-infrared LED on a ball or pad side of the fingertip and arranging a reflecting plate (e.g. , a mirror) on the fingernail side thereof.

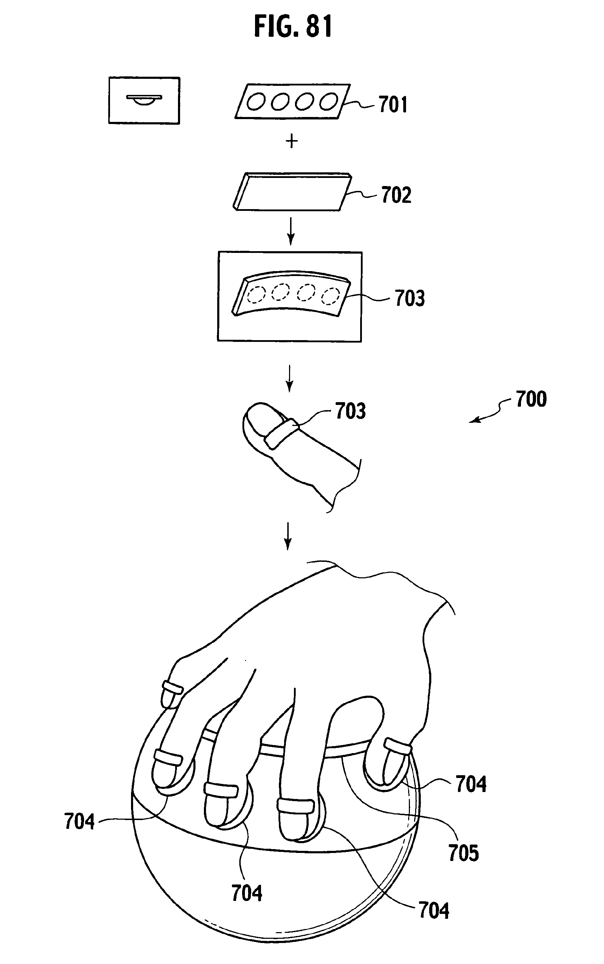

Fig. 81 shows a ninth embodiment of a fingertip stimulating apparatus according to the present invention.

A fingertip stimulating apparatus 700 shown therein employs a metal or metalized (plated) resin 701 as a reflecting plate, a slime gel 702 is bonded onto the metal and the resin 701, thereby forming an adhesive reflecting member 703. The adhesive reflecting member 703 is bonded to a position of a border of a fingernail of a finger, and a ball or pad side of the fingertip touches an irradiation surface of a near-infrared LED 704. A near-infrared ray can be thereby irradiated by the reflection method onto the position of the border of the fingernail of each fingertip as shown in Fig. 80(b).

The fingertip stimulating apparatus 700 also includes a blue-violet light emission ling 705, which is obtained by emitting a blue-violet light emitted from the blue-violet LED in the form of a ring using an appropriate prism.

### INDUSTRIAL APPLICABILITY

As described above, the fingertip stimulating apparatus according to the present invention is configured to include a fingertip arranging unit on which a fingertip of a hand is arranged, and a near-infrared generating member irradiating a near-infrared ray onto a region near a border of a fingernail of the fingertip arranged on the fingertip arranging unit. It is, therefore, possible to ensure stimulating the fingertips of the hand, to provide a safe and effective stimulus to the autonomic nerves, to control the body temperature, and for any one to handle the fingertip stimulating apparatus.

Furthermore, according to the present invention, the body temperature is regulated to accelerate increase of peripheral blood flow by effectively providing a visual stimulation as well as the finger stimulation.

## Claims

1. A fingertip stimulating apparatus comprising:
a fingertip arranging unit arranging a fingertip of a hand; and
a near-infrared generating member irradiating a near-infrared ray onto a region near a border of a fingernail of the fingertip arranged on the fingertip arranging unit.

2. A fingertip stimulating apparatus comprising:
a fingertip arranging unit arranging a fingertip of a hand;
a near-infrared generating member irradiating a near-infrared ray onto a region near a border of a fingernail of the fingertip arranged on the fingertip arranging unit; and
a blue-violet light generating member arranged visually recognizably, and emitting a blue-violet light.

3. A fingertip stimulating apparatus comprising:
a fingertip arranging unit arranging a fingertip of a hand;
a near-infrared generating member irradiating a near-infrared ray onto a region near a border of a fingernail of the fingertip arranged on the fingertip arranging unit; and
a light pulse generating member arranged visually recognizably, and emitting a light pulse.

4. A fingertip stimulating apparatus comprising:
a fingertip arranging unit arranging a fingertip of a hand;
a near-infrared generating member irradiating a near-infrared ray onto a region near a border of a fingernail of the fingertip arranged on the fingertip arranging unit; and
a blue-violet light generating member irradiating a blue-violet light onto a ball or pad-side region of the fingertip arranged on the fingertip arranging unit.

5. The fingertip stimulating apparatus according to claim 1, 2, 3 or 4, wherein
the near-infrared generating member includes a near-infrared LED intermittently turned on or off, and emitting the near-infrared ray.

6. The fingertip stimulating apparatus according to claim 1, 2, 3, 4 or 5, wherein
a wavelength of the near-infrared ray is preferably in a range of 820 nm to 920 nm, more preferably near 870 nm.

7. The fingertip stimulating apparatus according to claim 2, wherein
the blue-violet light generating member includes a blue-violet LED emitting the blue-violet light while being intermittently turned on or off.

8. The fingertip stimulating apparatus according to claim 4, wherein
the blue-violet light generating member includes a blue-violet LED emitting the blue-violet light while being continued to be turned on.

9. The fingertip stimulating apparatus according to claim 2, 4, 7 or 8, wherein
a wavelength of the blue-violet light is preferably in a range of 390 nm to 420 nm, more preferably near 405 nm.

10. The fingertip stimulating apparatus according to claim 3, wherein
the light pulse generating member emits the light pulse preferably at around 3 Hz.

11. The fingertip stimulating apparatus according to claim 1, 2 or 3, further comprising a vibration generating member vibrating the fingertip arranged on the fingertip arranging unit.

12. The fingertip stimulating apparatus according to claim 11, wherein
the vibration generating member produces a whizzing sound by crossing axis lines of vibrators generating vibrations.

13. The fingertip stimulating apparatus according to claim 1, 2, 3 or 4, wherein
the fingertip arranging unit includes a fingertip insertion unit causing the fingertip to be insertable from an opening formed in a surface of an apparatus main body into the apparatus main body.

14. The fingertip stimulating apparatus according to claim 13, wherein
the near-infrared generating member includes a near-infrared LED that is intermittently turned on or off to emit a near-infrared ray at a predetermined height from a bottom of the fingertip insertion unit, on a movable member that can be moved backward from an initial position at which the movable member enters the fingertip insertion unit by being pressed by the fingertip.

15. The fingertip stimulating apparatus according to claim 1, 2 or 3, wherein
the fingertip arranging unit includes
a fingertip insertion unit extending from an opening formed in a surface of an apparatus main body to an interior of the apparatus main body; and
an elastic member which is arranged in the fingertip insertion unit, into which the fingertip is insertable, which transmits the near-infrared ray, which is flexible, and which can be closely attached to the fingertip by pressurizing an airtight space between the elastic member and the fingertip insertion unit.

16. The fingertip stimulating apparatus according to claim 15, wherein
the near-infrared generating member includes a near-infrared LED generating the near-infrared ray while being intermittently turned on or off, and arranged at a predetermined height from a bottom of the fingertip insertion unit.

17. The fingertip stimulating apparatus according to claim 13 or 15, wherein
the apparatus main body is substantially spherical, and includes a total of five fingertip insertion units, the five fingertip insertion units being one fingertip insertion unit shared between a first finger of a left hand and a first finger of a right hand and four fingertip insertion units each shared between one of second to fifth fingers of the left hand and one of second to fifth fingers of the right hand.

18. The fingertip stimulating apparatus according to claim 4 or 13, wherein
the fingertip arranging unit includes a total of six fingertip insertion units in an apparatus main body, the six fingertip insertion units being four fingertip insertion units each shared between one of second to fifth fingers of a left hand and one of second to fifth fingers of a right hand, one fingertip insertion unit for a first finger of the left hand, and one fingertip insertion unit for a first finger of the right hand.

19. The fingertip stimulating apparatus according to claim 1, wherein
the fingertip arranging unit includes an elliptically cylindrical reflecting member, the fingertip arranging unit being one of focal regions of the elliptically cylindrical reflecting member, the near-infrared generating member being arranged in other focal region of the elliptically cylindrical reflecting member.
